# EUROPEAN PATENT SPECIFICATION

(11) **EP 1 656 183 B1**
(45) Date of publication and mention of the grant of the patent: **05.12.2007**
(21) Application number: 04743961.7
(22) Date of filing: 12.07.2004
(51) Int. Cl.: A61P 15/12

(54) **TREATMENT OF MALE SEXUAL DYSFUNCTION**
BEHANDLUNG DER MÄNNLICHEN SEXUELLEN DYSFUNKTION
TRAITEMENT DES DYSFONCTIONNEMENTS SEXUELS CHEZ L'HOMME

(30) Priority: 23.07.2003 IB 31722703
(43) Date of publication of application: 17.05.2006
(73) Proprietor: Pfizer Limited, Sandwich, Kent CT13 9NJ (GB); PFIZER INC., New York, N.Y. 10017 (US)
(72) Inventor: WAYMAN, Christopher P., Pfizer Global Res.&Devel., Sandwich, Kent CT13 9NJ (GB); RUSSELL, Rachel J., Pfizer Global Res.&Development, Sandwich, Kent CT13 9NJ (GB)
(74) Representative: Peter, Beate
(86) International application number: PCT/IB2004/002300
(87) International publication number: WO 2005/006899

(56) References cited:
- WO-A-01/58880
- WO-A-20/04037809

## Description

### Field of invention

The present invention relates the use of antagonists of vasopressin V1a receptors for the treatment of male sexual dysfunction, in particular ejaculatory disorders, such as premature ejaculation or rapid ejaculation.

The present invention also relates to a method of treatment of male sexual dysfunction, in particular ejaculatory disorders, such as premature ejaculation or rapid ejaculation.

The present invention also relates to assays to screen for compounds useful in the treatment of male sexual dysfunction, in particular ejaculatory disorders, such as premature ejaculation or rapid ejaculation.

### Introduction

Sexual dysfunction (SD) is a significant clinical problem which can affect both males and females. The causes of SD may be both organic as well as psychological. Organic aspects of SD are typically caused by underlying vascular diseases, such as those associated with hypertension or diabetes mellitus, by prescription medication and/or by psychiatric disease such as depression. Physiological factors include fear, performance anxiety and interpersonal conflict. SD impairs sexual performance, diminishes self-esteem and disrupts personal relationships thereby inducing personal distress. In the clinic, SD disorders have been divided into female sexual dysfunction (FSD) disorders and male sexual dysfunction (MSD) disorders (Melman et al 1999 J. Urology 161, 5-11). FSD is best defined as the difficulty or inability of a woman to find satisfaction in sexual expression. Male sexual dysfunction (MSD) is generally associated with either erectile dysfunction, also known as male erectile dysfunction (MED) and/or ejaculatory disorders such as premature ejaculation or rapid ejaculation, anorgasmia (unable to achieve orgasm) or desire disorders such as hypoactive sexual desire disorder (lack of interest in sex).

Premature ejaculation (PE) or rapid ejaculation is a relatively common sexual dysfunction in men. It has been defined in several different ways but the most widely accepted is the Diagnostic and Statistical Manual of Mental Disorders IV one which states:
"PE is a lifelong persistent or recurrent ejaculation with minimal sexual stimulation before, upon or shortly after penetration and before the patient wishes it. The clinician must take into account factors that affect duration of the excitement phase, such as age, novelty of the sexual partner or stimulation, and frequency of sexual activity. The disturbance causes marked distress of interpersonal difficulty."

The International Classification of Diseases 10 definition states:
"There is an inability to delay ejaculation sufficiently to enjoy lovemaking, manifest as either of the following: (1) occurrence of ejaculation before or very soon after the beginning of intercourse (if a time limit is required: before or within 15 seconds of the beginning of intercourse); (2) ejaculation occurs in the absence of sufficient erection to make intercourse possible. The problem is not the result of prolonged abstinence from sexual activity"

Other definitions which have been used include classification on the following criteria:
- Related to partner's orgasm
- Duration between penetration and ejaculation
- Number of thrust and capacity for voluntary control

Premature ejaculation/rapid ejaculation can be classified into various subgroups. It can be lifelong or acquired, it can be absolute or generalised (i.e. irrespective of partners and context) or situational (relative to a partner or context), etc. (see Jannini, E.A. et al (2002) J. Endocrinol. Invest. 25, 1006-1019) The vasopressin V1A antagonist is expected to work in all subgroups of premature ejaculation/rapid ejaculation.

Psychological factors may be involved in PE, with relationship problems, anxiety, depression, prior sexual failure all playing a role.

The estimated prevalence of PE is about 22-38% of the male population. Unlike male erectile dysfunction (MED), PE has no definite correlation with age. Taking an average prevalence of 30%, that would make an estimated 24 million sufferers in the US (males ages 18-65 was 80 million in 1995). There is little data on prevalence by severity. It is estimated that the operational definition of PE may apply to 5-10% of men, however, less than 0.2% present for treatment. The availability of an orally effective therapy is very likely to alter this situation.

Ejaculation is dependent on the sympathetic and parasympathetic nervous systems. Efferent impulses via the sympathetic nervous system to the vas deferens and the epididymis produce smooth muscle contraction, moving sperm into the posterior urethra. Similar contractions of the seminal vesicles, prostatic glands and the bulbouretheral glands increase the volume and fluid content of semen. Expulsion of semen is mediated by efferent impulses originating from the nucleus of Onuf in the spinal cord, which pass via the parasympathetic nervous system and cause rhythmic contractions of the bulbocavernous, ischiocavernous and pelvic floor muscles. Cortical control of ejaculation is still under debate in humans. In the rat the medial pre-optic area and the paraventricular nucleus of the hypothalamus seem to be involved in ejaculation.

There are at present no approved drugs available for treating PE. The most commonly off-label prescribed medications are the anti-depressants (for example clomipramine) and the selective serotonin re-uptake inhibitors (for example paroxetine and sertraline). These drugs are often not well accepted by patients because they are regarded as anti-depressants. They are used 'off-label', and though effective when used as required (i.e. 'prn'), due to their long pharmacokinetic Tₘₐₓ (time to maximum drug concentration in plasma following oral administration of the drug) they are likely to have a slow onset of action. Side-effects common to this class of drugs can be seen when used chronically. Behavioural therapy has been the other management tool but has not been very efficacious and has a high drop-out and relapse rate. New, more efficient therapies are required.

Thus, it is desirable to find new ways of treating male sexual dysfunction, in particular ejaculatory disorders such as premature ejaculation (PE).

The Role of Vasopressin in Sexual Behaviour

Ejaculation comprises two separate components - emission and ejaculation. Emission is the deposition of seminal fluid and sperm from the distal epididymis, vas deferens, seminal vesicles and prostrate into the prostatic urethra. Subsequent to this deposition is the forcible expulsion of the seminal contents from the urethral meatus. Ejaculation is distinct from orgasm, which is purely a cerebral event. Often the two processes are coincidental.

A pulse of oxytocin in peripheral serum accompanies ejaculation in mammals. In man oxytocin but not vasopressin plasma concentrations are significantly raised at or around ejaculation. Oxytocin does not induce ejaculation itself; this process is 100% under nervous control via α1-adrenoceptor/sympathetic nerves originating from the lumbar region of the spinal cord. The systemic pulse of oxytocin may have a direct role in the peripheral ejaculatory response. It could serve to modulate the contraction of ducts and glandular lobules throughout the male genital tract, thus influencing the fluid volume of different ejaculate components for example. Oxytocin released centrally into the brain could influence sexual behaviour, subjective appreciation of arousal (orgasm) and latency to subsequent ejaculation. The occurrence of ejaculation in males is critically dependent on tactile stimulation of the external genitalia.

It is well documented that the levels of circulating oxytocin increase during sexual stimulation and arousal, and peak during orgasm in both men and women. Murphy et al (Acta. Anat. Basel (1987) 128: 76-79) measured the plasma oxytocin and arginine vasopressin (AVP) concentrations in men during sexual arousal and ejaculation and found that plasma AVP but not oxytocin significantly increased during sexual arousal. However, at ejaculation, mean plasma oxytocin rose about five-fold and fell back to basal concentrations within 30 minutes, while AVP had already returned to basal levels at the time of ejaculation and remained stable thereafter. More detailed recent studies investigating the specificity of the neuroendocrine response to orgasm during sexual arousal in men have shown no change in plasma vasopressin levels during sexual arousal or orgasm/ejaculation (Kruger TH et al (2003) J Endocrinol. 177(1):57-64).

This raises the question as to whether vasopressin is involved in the male arousal/orgasm process at all. Stoneham et al (J Endocrinology (1985); 107(1)) found changes in oxytocin levels in rats and rabbits but again there was no effect on vasopressin plasma levels further questioning a role of vasopressin in copulatory behaviour. Animal data investigating the effects of oxytocin and vasopressin during sexual behaviour in freely moving rats demonstrate the oxytocin, but not vasopressin concentrations, are elevated during coitus (Hughes et al (1987) Brain Res, 414(1):133-137).

Vasopressin receptors have been identified in urogenital organs associated with ejaculation. - It has been suggested that vasopressin may have a role in regulating sperm transport from the epididymis, or alternatively by influencing seminiferous tubule, vas deferens and prostate contractility. However the role of vasopressin and vasopressin receptors in ejaculation is unclear.

Immunoreactive arginine vasopressin (AVP) and effects of AVP on the human vas deferens has been investigated by Andersson (J Urol. (1988) 140(5):1054-7). They suggest that circulating AVP is taken up and accumulated by the human vas deferens, and/or that AVP is synthesized locally. Contractile effects of vasopressin on human vas deferens have been demonstrated by Medina et al (Eur J Pharmacol. (1998): 355: 41-49) and these appear to be mediated by V1 vasopressin receptors. Medina et al demonstrated also that vasopressin strongly potentiates contractions of human vas deferens elicited by adrenergic stimulation. Both the direct and indirect effects of vasopressin appear to be mediated by vasopressin V1 receptor stimulation. The physiological role of the AVP occurring in the human vas deferens remains to be established.

Arginine vasopressin has been shown to raise prostatic tone and elicit contractions of mammalian prostate tissue. The potency of vasopressin is significantly less than that observed for oxytocin. Bodanszky et al ((1992) Eur. J. Pharmacol. 216, 311-313) suggest a physiological role for arginine vasotocin, oxytocin and arginine vasopressin in prostatic smooth muscle contraction and possibly also in other aspects of male reproductive function.

There is a high density of vasopressin receptors in the epithelial cells of porcine seminal vesicles. The authors demonstrate that a very high affinity (0.2 nM), low capacity (14 fmoles/mg protein) class of vasopressin receptors is present in human seminal vesicles, having pharmacologic characteristics similar to the V1 subtype of vasopressin receptors. The density of the vasopressin receptors present in human seminal vesicles is inversely correlated with patient age and they conclude that this is consistent with a physiologic role for vasopressin in the regulation of accessory sex gland activity (Maggi et al (1989) J Androl. 10(5):393-400). ,

The only in vivo studies looking at the effects of vasopressin on ejaculation have been performed in sheep and rabbits. Nicholson et al (J Reprod Fertil. (1999) 117(2):299-305) have investigated the effects of oxytocin and vasopressin on sperm transport from the cauda epididymis in sheep. They found that vasopressin did not increase the number or concentration of spermatozoa in the fluid and appeared to decrease fluid output. There were no reported effects on time to ejaculation. In rabbits Agmo (J Reproduction and Fertility 45(2); 243-248) reported that vasopressin increased the number of spermatozoa in the ejaculates. There was no change in the time it took the rabbit to achieve orgasm ie ejaculation latency.

Selectivity over Oxytocin receptors will reduce the potential for treatment-related male erectile dysfunction (MED).

As detailed in Gimpl and Fahrenholz (Physiological Reviews (2001), 81 (2) 629-683), oxytocin has been found to be one of the most potent agents to induce penile erection in rats, rabbits and monkeys. In addition, central administration of oxytocin is claimed to reduce the latency to achieve ejaculation and to shorten the post-ejaculatory interval. Likewise, Meston et al (Arch. Gen Psychiatry (2000) Vol 57) states that in male animals, oxytocin facilitates penile erections when injected into specific areas of the brain (i.e. periventricular nucleus of the hypothalamus) and shortens the ejaculation latency and post-ejaculation interval when injected either centrally or peripherally.

It has been well documented within the art that the administration of the oxytocin receptor antagonist, vasotocin, significantly reduces non-contact penile erections (see, for example, Melis et al (Neuroscience Letters 265 (1999) 171-174). In addition, intracerebroventricular (ICV) injection of the oxytocin antagonist vasotocin was shown in Argiolas et al (European Journal of Pharmacology 149 (1988) 389-392) to impair sexual performance in experienced male rats in the presence of a receptive female, with the abolishment of ejaculation (probably caused by a decreased intromission frequency). The decrease in intromission frequency was thought to reflect a decreased capacity of the animals to achieve penile erection, as the oxytocin antagonist was found to prevent penile erection.

Therefore, it is highly surprising to find that vasopressin V1a receptor antagonists can delay ejaculation, and are thus useful in treating ejaculation disorders, such as premature ejaculation or rapid ejaculation.

### Aspects of the Invention

A seminal finding of the invention is the surprising result that by administering a vasopressin V1a receptor antagonist, an increase in latency to ejaculation can be achieved. Thus, we have shown that a vasopressin V1a receptor antagonist can be used for the treatment of ejaculatory disorders, in particular premature ejaculation or rapid ejaculation. This may be achieved by increasing ejaculatory latency, preferably by restoring ejaculatory latency to near normal levels.

The treatment of ejaculatory disorders, in particular premature ejaculation or rapid ejaculation, with a vasopressin V1a receptor antagonist allows the treatment thereof whilst maintaining the patient's sexual drive. The term "sexual drive" as used herein means libido or sexual desire.

Thus, compounds according to the present invention preferably comprise the unexpected advantage of maintaining erectogenic mechanisms, in particular penile erection, and/or sexual drive, as compared with known non-selective vasopressin/oxytocin antagonists (which are also more likely to cause erectile dysfunction), and will not be associated with some of the less desirable CNS effects of SSRI'S, such as sleep disturbances.

Therefore the invention relates to vasopressin V1 a receptor antagonists for use in the treatment of ejaculatory disorders, preferably premature ejaculation or rapid ejaculation. The invention also relates to the use of vasopressin V1a receptor antagonists for the manufacture of a medicament for the treatment of ejaculatory disorders, preferably premature ejaculation or rapid ejaculation. The invention also relates to a method of treatment of ejaculatory disorders, preferably premature ejaculation or rapid ejaculation, with a vasopressin V1a receptor antagonist. One aspect of the invention is therefore a method of treating ejaculatory disorders, preferably premature ejaculation or rapid ejaculation, comprising the administration to a patient in need of such treatment of an effective amount of a vasopressin V1a receptor antagonist. The term "ejaculatory disorders" includes premature ejaculation or rapid ejaculation. The term "treatment" includes the palliative, curative and prophylactic treatment of ejaculatory disorders, preferably premature ejaculation or rapid ejaculation, complications arising from ejaculatory disorders, preferably premature ejaculation or rapid ejaculation.

The vasopressin V1a receptor antagonist preferably will have an IC₅₀ in a ligand binding assay of less than 100nM, more preferably an IC₅₀ of less than 10nM, even more preferably an IC₅₀ of less than 5nM. The IC₅₀ may be measured in a ligand binding assay, e.g. as described in Example 2, or in a functional assay, e.g. measuring a transient increase in intracellular calcium (see, for example, Example 3).

Preferably the vasopressin V1 a receptor antagonists will be at least 10 fold selective over oxytocin receptors, more preferably at least 100 fold selective over oxytocin receptors. Preferably the vasopressin V1 a receptor antagonists will be at least 10 fold selective over vasopressin V2 receptors, more preferably at least 100 fold selective over vasopressin V2 receptors. Preferably the vasopressin V1 a receptor antagonists will be at least 10 fold selective over vasopressin V1b receptors, more preferably at least 100 fold selective over vasopressin V1 b receptors.

Another aspect of the invention is the use of a compound of formula **(Ia)**, 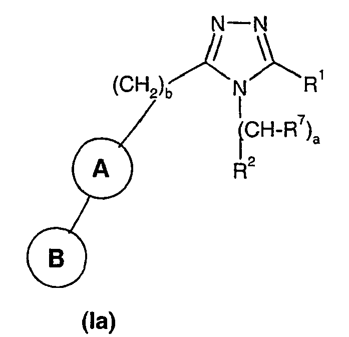 or a pharmaceutically acceptable salt or solvate thereof, wherein
R¹ represents C₁-C₆ alkyl, -(CH₂)_{c}-[C₃-C₈ cycloalkyl]-, -(CH₂)_{c}-W or -(CH₂)_{c}-Z-(CH₂)_{d}-W;
R² represents a phenyl group, optionally fused to a 5- or 6- membered aryl or heterocyclic group which may contain one or more heteroatoms selected from N, O or S; the phenyl group and the optionally fused group being optionally substituted with one or more groups independently selected from the list defined below;
Ring **A** represents a 4-, 5- or 6- membered saturated heterocyclic group containing at least one N;
Ring **B** represents a phenyl group or het¹, each group being optionally substituted with one or more groups independently selected from the list defined below;
het¹ represents a 4-, 5- or 6- membered saturated, or unsaturated, heterocyclic group containing at least one N (but which may also contain one or more O or S atoms);
R⁷ independently represents H, C₁-C₆ alkyl, OR³, -(CH₂)ₑ-R³ or -(CH₂)_{f}-O-(CH₂)ₑ-R³;
W represents a phenyl group, NR⁴R⁵ or het², the phenyl group being optionally substituted with one or more groups independently selected from halogen, CF₃, OCF₃, R³, OR³, CO₂R³, CONR⁴R⁵, CN, SO₂NR⁴R⁵ and NR³SO₂Me;
het² represents a 4-, 5-, 6- or 7- membered saturated, or unsaturated, heterocyclic group containing at least one N (but which may also contain one or more O or S atoms), optionally substituted with one or more groups independently selected from the list defined below;
Z represents O or S(O)_{g};
g represents 0, 1 or 2;
het³ represents a 4-, 5-, 6- or 7- membered saturated or unsaturated heterocyclic group containing at least one N (but which may also contain one or more O or S atoms), optionally substituted with one or more groups independently selected from the list defined below;
at each occurrence R³ and R⁶ independently represent H, C₁-C₆ alkyl optionally substituted by Y, -(CH₂)_{g}-[C₃-C₈ cycloalkyl], phenyl, benzyl, pyridyl or pyrimidyl;
Y independently represents a phenyl group, NR⁴R⁵ or het³, the phenyl group being optionally substituted with one or more groups independently selected from halogen, CF₃, OCF₃, R⁴, OR⁴, CO₂R⁴, CONR⁴R⁵, CN, SO₂NR⁴R⁵, NR⁴SO₂Me and -NR⁴R⁵;
at each occurrence R⁴ and R⁵ independently represent H, C₁-C₆ alkyl, -(CH₂)_{g}-[C₃-C₈ cycloalkyl], phenyl, benzyl, pyridyl or pyrimidyl; or R⁴ and R⁵ together with the N atom to which they are attached represent a heterocyclic group of from 3 to 8 atoms;
substituents for R², Ring **B,** het¹, het² and het³ are independently selected from the following list: halogen, CF₃, OCF₃, R³, -(CH₂)ₑ-OR³, -(CH₂)ₑ-CO₂R³, -(CH₂)ₑ-CONR⁴R⁵,-(CH₂)e-CN, -(CH₂)ₑ-SO₂NR⁴R⁵, -(CH₂)ₑ-NR³SO₂Me, -(CH₂)ₑ-COR³, -(CH₂)ₑ-OCOR³,-(CH₂)ₑ-NHCOR³, -(CH₂)ₑ-NR³COR⁶ and -(CH₂)ₑNR⁴R⁵;
a and b independently represent 0 or 1;
c, d, e and g independently represent 0, 1, 2, 3 or 4;
f independently represents 1, 2, 3 or 4;
provided that a + b cannot equal 0; and
provided that when R¹ represents -(CH₂)_{c}-Z-(CH₂)_{d}-W and W represents NR⁴R⁵ or any N linked heterocyclic group then d must not be 0 or 1; and
provided that when R² represents a phenyl group substituted by a group of formula - (CH₂)ₑOR³, -(CH₂)ₑ-CO₂R³ or -(CH₂)ₑOCOR³; or
het¹ and/or het² are substituted by a group of formula -(CH₂)ₑOR³, -(CH₂)ₑ-CO₂R³ or - (CH₂)ₑOCOR³; or
when R⁷ represents -OR³ or -(CH₂)_{f}-O-(CH₂)ₑ-R³ and e is 0; or
when W represents a phenyl group substituted with -OR³ or -CO₂R³;
and R³ represents an alkyl group substituted with Y, and Y represents NR⁴R⁵ or an N-linked het³;
then R³ must represent C₂-C₆ alkyl substituted with Y,
in the manufacture of a medicament for the treatment of premature ejaculation.

Preferred groups of compounds for use with the present invention are those in which:
(i) R¹ is C₃-C₆ cycloalkyl or C₁-C₄ alkyl, and more preferably methyl, i-propyl or n-butyl;
(ii) R¹ is -(CH₂)_{c}-W or -(CH₂)_{c}-Z-(CH₂)_{d}-W;
(iii) R² is a phenyl group optionally substituted with one or more groups selected from halogen or -(CH₂)ₑ-OR³;
(iv) ring **A** is selected from piperidinyl, piperazinyl, azetidinyl or pyrrolidinyl and more preferably it is piperidinyl or piperazinyl;
(v) ring **B** is a phenyl group substituted groups one or more groups selected from halogen, CF₃, OCF₃, R³, -(CH₂)ₑ-OR³ and CN;
(vi) ring **B** is an unsubstituted phenyl group;
(vii) ring **B** is het¹
(viii) R⁷ is C₁-C₄ alkyl, more preferably it is C₁-C₄ straight chain alkyl and most preferably it is methyl or ethyl;
(ix) R⁷ is CH₂OH;
(x) W is a halo substituted phenyl group;
(xi) W is NR⁴R⁵, preferably it is selected from NHMe, NMe₂, NEt₂, N(iPr)₂, or N(nPr)₂;
(xii) W is C₁-C₆ alkyl, preferably methyl or ethyl;
(xiii) W is CO₂[C₁-C₆ alkyl], preferably CO₂^{t}Bu;
(xiv) W is het²or het³;
(xv) W is OMe;
(xvi) W is CONR⁴R⁵;
(xvii) W is halogen, preferably chloro or fluoro
(xviii) Z is O;
(xix) R³ and R⁶ are independently C₁₋₄ alkyl, more preferably unsubstituted C₁₋₄ alkyl, even more preferably methyl or tert-butyl;
(xx) R³ and R⁶ are independently H or benzyl;
(xxi) R⁴ and R⁵ are independently selected from H, methyl, ethyl, n-propyl or i-propyl;
(xxii) R⁴ and R⁵ together with the nitrogen to which they are attached form a heterocycle preferably selected from piperidinyl, 2-oxa-5-aza-bicyclo[2.2.1]heptanyl, piperazinyl, azetidinyl, imidazolyl, pyrazolyl, triazolyl, morpholinyl and pyrrolidinyl;
(xxiii) R⁴ and R⁵ are independently selected from SO₂Me or benzyl;
(xxiv) R⁴ and R⁵ are independently CH₂CO₂-[C₁-C₆alkyl], preferably CH₂CO₂^{t}Bu;
(xxv) R⁴ and R⁵ are independently C₁-C₆alkyl substituted by C₁-C₆alkyloxy, preferably C₂-C₃ alkyl substituted by methoxy;
(xxv) het¹ is selected from optionally substituted pyridinyl, pyrimidinyl, pyrazinyl, pyridazinyl, triazinyl, triazolyl, tetrazolyl, pyrrolyl, pyrazolyl, imidazolyl, oxazolyl, isoxazolyl, thiazolyl, piperidinyl, piperazinyl, azetidinyl, morpholinyl, 2-oxa-5-aza-bicyclo[2.2.1.]heptanyl or pyrrolidinyl, and more preferably selected from pyridinyl, pyrazinyl or pyrimidinyl, optionally substituted by any one of R³;
(xxvi) het² is selected from substituted or unsubstituted pyridinyl, pyrimidinyl, pyrazinyl, pyridazinyl, triazinyl, triazolyl, tetrazolyl, pyrrolyl, pyrazolyl, imidazolyl, oxazolyl, isoxazolyl, thiazolyl, piperidinyl, piperazinyl, N-methyl piperazinyl, azetidinyl, morpholinyl, 2-oxa-5-aza-bicyclo[2.2.1]heptanyl or pyrrolidinyl and more preferably selected from imidazolyl, piperidinyl, piperazinyl, N-methyl piperazinyl, azetidinyl, morpholinyl, 2-oxa-5-aza-bicyclo[2.2.1]heptanyl or pyrrolidinyl. Even more preferably it is pyridinyl;
(xxvii) het³ is selected from substituted or unsubstituted tetrahydro-furanyl or tetrahydropyranyl;
(xxviii) substituents for R², Ring **B,** het¹, het², het³ and het⁴ are independently CF₃, R³, - (CH₂)ₑ-OR³, -(CH₂)ₑ-CO₂R³, -(CH₂)₃-CN, -(CH₂)ₑ-SO₂Me, -(CH₂)ₑ-COR³;
(xxix) Z is O or S;
(xxx) a is 1;
(xxxi) b is 0;
(xxxii) c is selected from 0, 1 or 2. More preferably it is selected from 0 or 1;
(xxiii) e is selected from 0, 1 or 2 and more preferably selected from 0 or 1, even more preferably it is 0;
(xxxiv) d is selected from 0, 1, 2 or 3 and more preferably it is selected from 0 to 2;
(xxxv) f is selected from 1 or 2, preferably it is 1;
(xxvi) g is 0.

Preferred compounds according to the present invention are:
(*S*)-4-[5-Butyl-4-(1-phenyl-ethyl)-4*H*-[1,2,4]triazol-3-yl]-3,4,5,6-tetrahydro-2*H-*[1,2']bipyridinyl;
2-[4-(4-Benzyl-5-isobutyl-4*H*-[1,2,4]triazol-3-yl)-piperidin-1-yl]-pyrimidine;
(*S*)-4-[5-Methyl-4-(1-phenyl-ethyl)-4*H*-[1,2,4]triazol-3-yl]-3,4,5,6-tetrahydro-2*H-*[1,2']bipyridinyl;
4-[4-Benzyl-5-butyl-4*H*-[1,2,4]triazol-3-yl]-3,4,5,6-tetrahydro-2*H*[1,2']bipyridinyl;
2-[4-(4-Benzyl-5-isopropyl-4*H*-[1,2,4]triazol-3-yl)-piperidin-1-yl]-pyrimidine;
2-[4-(4-Benzyl-5-cyclopropyl-4*H*-[1,2,4]triazol-3-yl)-piperidin-1-yl]-pyrimidine;
(*S*)-2-{4-[5-Methyl-4-(1-phenyl-propyl)-4*H*-[1,2,4]triazol-3-yl)-piperidin-1-yl]-pyrimidine;
2-[4-(4-Benzyl-5-propyl-4*H*-[1,2,4]triazol-3-yl)-piperidin-1-yl]-pyrimidine;
2-{ 4-[4-Benzyl-5-(2-chloro-phenoxymethyl)-4*H*-[1,2,4]triazol-3-yl]-piperidin-1-yl}-pyrimidine;
2-[4-(4-Benzyl-5-butyl-4*H* [1,2,4]triazol-3-yl)-piperidin-i-yl]-pyrimidine;
(*S*)-2-{4-[5-Methyl-4-(1-phenyl-ethyl)-4*H*-[1,2,4]triazol-3-yl)-piperidin-1-yl]-pyrimidine;
2-{4-[4-Benzyl-5-(4-fluoro-phenoxymethyl)-4*H*-[1,2,4]triazol-3-yl]-piperidin-1-yl}-pyrimidine;
2-{4-[5-Methyl-4-(3-methyl-benzyl)-4*H*-[1,2,4]triazol-3-yl)-piperidin-1-yl]-pyrimidine;
(*S*)-2-(4-[5-Methyl-4-(1-phenyl-ethyl)-4*H*-[1,2,4]triazol-3-ylmethyl]-piperidin-1-yl)-pyrimidine;
2-{4-[4-(3-Fluoro-benzyl)-5-methyl-4*H*-[1,2,4]triazol-3-yl)-piperidin-1-yl]-pyrimidine;
4-(4-Benzyl-5-morpholin-4-ylmethyl-4*H*-[1,2,4]triazol-3-yl)-3,4,5,6-tetrahydro-2*H-*[1,2']bipyridinyl;
4-(4-Benzyl-5-benzyloxymethyl-4*H*-[1,2,4]triazol-3-yi)-3,4,5,6-tetrahydro-2*H-*[1,2']bipyridinyl;
4-(4-Benzyl-5-methyl-4*H*-[1,2,4]triazol-3-yl)-3,4,5,6-tetrahydro-2*H*-[1,2']bipyridinyl;
(*R*)-2-[3-Methyl-5-(1-pyrimidin-2-yl-piperidin-4-yl)-[1,2,4]triazol-4-yl]-2-phenyl-ethanol;
2-[4-(4-Benzyl-5-methyl-4*H*-[1,2,4]triazol-3-yl)-piperidin-1-yl]-4-methyl-pyrimidine;
2-[4-(4-Benzyl-5- methyl-4H-[1,2,4]triazol-3-yl)-piperidin-1-yl]-pyrimidine;
4-(4-Benzyl-5-methyl-4H-[1,2,4]triazol-3-yl)-1-phenyl-piperidine;
2-[4-(4-Benzyl-5-methyl-4H[1,2,4]triazol-3-yl)-piperidin-1-yl]-pyrazine;
4-(4-Benzyl-5-piperidin-1-ylmethyl-4H-[1,2,4]triazol-3-yl)-3,4,5,6-tetrahydro-2H-[1,2']bipyridinyl;
(*S*)-4-[4-(1-Phenyl-ethyl)-5-piperidin-1-ylmethyl-4*H*[1,2,4]triazol-3-yl]-3,4,5,6-tetrahydro-2*H*-[1,2']bipyridinyl;
4-[4-Benzyl-5-(4-methoxy-piperidin-1-ylmethyl)-4*H*[1,2,4]triazol-3-yl]-3,4,5,6-tetrahydro-2*H*-[1,2']bipyridinyl;
(*S*)-4-[5-(4-Methoxy-piperidin-1-ylmethyl)-4-(1-phenyl-ethyl)-4*H*-[1,2,4]triazol-3-yl]-3,4,5,6-tetrahydro-2*H*-[1,2']bipyridinyl;
4-[4-Benzyl-5-(3,4,5,6-tetrahydro-2H-[1,2']bipyridinyl-4-yl)-4H-[1,2,4]triazol-3-ylmethyl]-piperazine-1-carboxylic acid benzyl ester;
4-[4-Benzyl-5-(2-morpholin-4-yl-ethoxymethyl)-4H-[1,2,4]triazol-3-yl]-3,4,5,6-tetrahydro-2H-[1,2']bipyridinyl.
4-[4-Benzyl-5-{(3*R*)-3-methoxy-pyrrolidin-1-ylmethyl)-4H-[1,2,4]triazol-3-yl]-3,4,5,6-tetrahydro-2H-[1,2']bipyridinyl
4-[4-Benzyl-5-{(3*S*)-3-methoxy-pyrrolidin-1-ylmethyl)-4H-[1,2,4]triazol-3-yl]-3,4,5,6-tetrahydro-2H-[1,2']bipyridinyl
1-[4-Benzyl-5-(3,4,5,6-tetrahydro-2H-[1,2']bipyridinyl-4-yl)-4H-[1,2,4]triazol-3-ylmethyl]-pyrrolidin-3-ol
4-(4-Benzyl-5-pyrrolidin-1-ylmethyl-4H-[1,2,4]triazol-3-yl)-3,4,5,6-tetrahydro-2H-[1,2']bipyridinyl
4-[4-Benzyl-5-(2-oxa-5-aza-bicyclo[2.2.1]hept-5-ylmethyl)-4H-[1,2,4]triazol-3-yl]-3,4,5,6-tetrahydro-2H-[1,2']bipyridinyl
4-[4-Benzyl-5-(4-methoxy-piperidin-1-ylmethyl)-4H-[1,2,4]triazol-3-yl]-3,4,5,6-tetrahydro-2H-[1,2']bipyridinyl
4-[4-(4-Fluoro-benzyl)-5-methyl-4H-[1,2,4]triazol-3-yl]-3,4,5,6-tetrahydro-2H-[1,2']bipyridinyl
4-[4-(3-Methoxy-benzyl)-5-methyl-4H-[1,2,4]triazol-3-yl]-3,4,5,6-tetrahydro-2H-[1,2']bipyridinyl
4-[5-Methyl-4-(3-methyl-benzyl)-4H-[1,2,4]triazol-3-yl]-3,4,5,6-tetrahydro-2H-[1,2']bipyridinyl
4-[4-(3-Chloro-benzyl)-5-methyl-4H-[1,2,4]triazol-3-yl]-3,4,5,6-tetrahydro-2H-[1,2']bipyridinyl
N-Benzyl-2-[4-benzyl-5-(3,4,5,6-tetrahydro-2H-[1,2']bipyridinyl-4-yl)-4H-[1,2,4]triazol-3-yl]-acetamide
2-[4-Benzyl-5-(3,4,5,6-tetrahydro-2H-[1,2']bipyridinyl-4-yl)-4H-[1,2,4]triazol-3-ylmethoxy]-ethylamine
[4-Benzyl-5-(3,4,5,6-tetrahydro-2H-[1,2']bipyridinyl-4-yl)-4H-[1,2,4]triazol-3-ylmethyl]-ethyl-amine
[4-Benzyl-5-(3,4,5,6-tetrahydro-2H-[1,2']bipyridinyl-4-yl)-4H-[1,2,4]triazol-3-ylmethyl]-(2-methoxy-ethyl)-amine
[4-Benzyl-5-(3,4,5,6-tetrahydro-2H-[1,2']bipyridinyl-4-yl)-4H-[1,2,4]triazol-3-ylmethyl]-(3-methoxy-propyl)-amine
1-{4-[4-Benzyl-5-(3,4,5,6-tetrahydro-2H-[1,2']bipyridinyl-4-yl)-4H-[1,2,4]triazol-3-ylmethyl]-piperazin-1-yl}-ethanone
4-[4-Benzyl-5-(4-methanesulfonyl-piperazin-1-ylmethyl)-4H-[1,2,4]triazol-3-yl]-3,4,5,6-tetrahydro-2H-[1,2]bipyridinyl
N-[4-Benzyl-5-(3,4,5,6-tetrahydro-2H-[1,2']bipyridinyl-4-yl)-4H-[1,2,4]triazol-3-ylmethyl]-methanesulfonamide
[4-Benzyl-5-(3,4,5,6-tetrahydro-2H-[1,2']bipyridinyl-4-yl)-4H-[1,2,4]triazol-3-ylmethyl]-(2-methoxy-ethyl)-methyl-amine
[4-Benzyl-5-(3,4,5,6-tetrahydro-2H-[1,2']bipyridinyl-4-yl)-4H-[1,2,4]triazol-3-ylmethyl]-(3-methoxy-propyl)-methyl-amine
4-(4-Benzyl-5-morpholin-4-ylmethyl-4H-[1,2,4]triazol-3-yl)-3'-methyl-3,4,5,6-tetrahydro-2H-[1,2']bipyridinyl
4-(4-Benzyl-5-morpholin-4-ylmethyl-4H-[1,2,4]triazol-3-yl)-3'-trifluoromethyl-3,4,5,6-tetrahydro-2H-[1,2']bipyridinyl
4-(4-Benzyl-5-morpholin-4-ylmethyl-4H-[1,2,4]triazol-3-yl)-3,4,5,6-tetrahydro-2H-[1,2']bipyridinyl-3'-carbonitrile
4-(4-Benzyl-5-morpholin-4-ylmethyl-4H-[1,2,4]triazol-3-yl)-3,4,5,6-tetrahydro-2H-[1,2']bipyridinyl-3'-carboxylic acid amide
(S)-4-[4-(1-Phenyl-ethyl)-5-(4-pyridin-2-yl-piperazin-1-ylmethyl)-4H-[1,2,4]triazol-3-ylmethyl]-morpholine trihydrochloride
(S)-4-[4-(1-Phenyl-ethyl)-5-(4-pyrimidin-2-yl-piperazin-1-ylmethyl)-4H-[1,2,4]triazol-3-ylmethyl]-morpholine trihydrochloride
1-[4-Benzyl-5-(1-pyrimidin-2-yl-piperidin-4-yl)-4H-[1,2,4]triazol-3-ylmethyl]-piperidin-3-ol
(R)- 2-[4-Benzyl-5-(3,4,5,6-tetrahydro-2H-[1,2']bipyridinyl-4-yl)-4H-[1,2,4]triazol-3-yl]-pyrrolidine-1-carboxylic acid tert-butyl ester
(R)-4-[4-Benzyl-5-(tetrahydro-furan-3-yloxymethyl)-4H-[1,2,4]triazol-3-yl] 3,4,5,6-tetrahydro-2H-[1,2']bipyridinyl
(S)-4-[4-Benzyl-5-(tetrahydro-furan-3-yloxymethyl)-4H-[1,2,4]triazol-3-yl] 3,4,5,6-tetrahydro-2H-[1,2']bipyridinyl
{[4-Benzyl-5-(3,4,5,6-tetrahydro-2H-[1,2']bipyridinyl-4-yl)-4H-[1,2,4]triazol-3-ylmethyl]-methyl-amino}-acetic acid tert-butyl ester
4-[4-Benzyl-5-(tetrahydro-pyran-4-ylmethyl)-4H-[1,2,4]triazol-3-yl]-3,4,5,6-tetrahydro-2H-[1,2']bipyridiny
4-[4-Benzyl-5-(tetrahydro-furan-2-yl)-4H-[1,2,4]triazol-3-yl]-3,4,5,6-tetrahydro-2H-[1,2']bipyridinyl
4-(4-Benzyl-5-ethoxymethyl-4H-[1,2,4]triazol-3-yl)-3,4,5,6-tetrahydro-2H-[1,2']bipyridinyl
4-[4-Benzyl-5-(2-methoxy-ethoxymethyl)-4H-[1,2,4]triazol-3-yl]-3,4,5,6-tetrahydro-2H-[1,2']bipyridinyl
[4-Benzyl-5-(3,4,5,6-tetrahydro-2H-[1,2']bipyridinyl-4-yl)-4H-[1,2,4]triazol-3-ylmethoxy]-acetic acid tert-butyl ester
N-Benzyl-2-[4-benzyl-5-(3,4,5,6-tetrahydro-2H-[1,2']bipyridinyl-4-yl)-4H-[1,2,4]triazol-3-ylmethoxy]-acetamide
4-(4-Benzyl-5-methylsulfanylmethyl-4H-[1,2,4]triazol-3-yl)-3,4,5,6-tetrahydro-2H-[1,2']bipyridinyl
4-(4-Benzyl-5-pyrazol-1-ylmethyl-4H-[1,2,4]triazol-3-yl)-3,4,5,6-tetrahydro-2H-[1,2']bipyridinyl
4-(4-Benzyl-5-[1,2,3]triazol-2-ylmethyl-4H-[1,2,4]triazol-3-yl)-3,4,5,6-tetrahydro-2H-[1,2']bipyridinyl
4-(4-Benzyl-5-[1,2,3]triazol-1-ylmethyl-4H-[1,2,4]triazol-3-yl)-3,4,5,6-tetrahydro-2H-[1,2']bipyridinyl
4-[4-Benzyl-5-(pyridin-4-yloxymethyl)-4H-[1,2,4]triazol-3-yl]-3,4,5,6-tetrahydro-2H-[1,2']bipyridinyl, or pharmaceutically acceptable derivatives thereof, in particular pharmaceutically acceptable salts thereof.

Of particular interest are the following compounds:
4-[4-Benzyl-5-butyl-4*H*-[1,2,4]triazol-3-yl]-3,4,5,6-tetrahydro-2*H*-[1,2']bipyridinyl
4-(4-Benzyl-5-morpholin-4-ylmethyl-4*H*-[1,2,4]triazol-3-yl)-3,4,5,6-tetrahydro-2*H-*[1,2']bipyridinyl
4-(4-Benzyl-5-benzyloxymethyl-4*H*-[1,2,4]triazol-3-yl)-3,4,5,6-tetrahydro-2*H-*[1,2']bipyridinyl
4-(4-Benzyl-5-piperidin-1-ylmethyl-4H-[1,2,4]triazol-3-yl)-3,4,5,6-tetrahydro-2H-[1,2']bipyridinyl
(*S*)-4-[4-(1-Phenyl-ethyl)-5-piperidin-1-ylmethyl-4*H*-[1,2,4]triazol-3-yl]-3,4,5,6-tetrahydro-2*H*-[1,2']bipyridinyl
4-[4-Benzyl-5-(4-methoxy-piperidin-1-ylmethyl)-4*H*-[1,2,4]triazol-3-yl]-3,4,5,6-tetrahydro-2*H*-[1,2']bipyridinyl, or pharmaceutically acceptable derivatives thereof, in particular pharmaceutically acceptable salts thereof.

These compounds were disclosed and their synthesis described in WO 04/037809.

Yet another aspect of the invention is the use of a compound of formula (I) 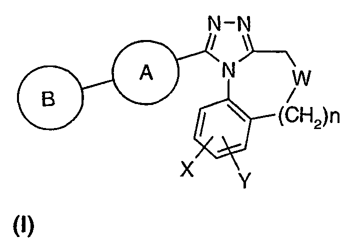 or a pharmaceutically acceptable salt or solvate thereof, wherein
W is O, S, or NR¹
R¹ represents H, C₁₋₆ alkyl, -(CH₂)ₐ-[C₃₋₈ cycloalkyl], phenyl, benzyl, pyridyl, pyrimidyl, - COR², -CO₂R², -CO-(CH₂)ₐ-NR²R³, -SO₂R², -(CH₂)_{b}-OR², -(CH₂)_{b}-NR²R³, or a saturated heterocycle of from 3 to 8 atoms containing one or more heteroatoms selected from O, N and S;
X and Y independently represent H, halogen, OH, CF₃, OCF₃, R⁴, -(CH₂)_{d}-CONR⁴R⁵,-(CH₂)_{d}-CN, -(CH₂)_{d}-SO₂NR⁴R⁵, -(CH₂)_{d}-NR⁴SO₂Me, -(CH₂)_{d}-COR⁴, -(CH₂)_{d}-OCOR⁴,-(CH₂)_{d}-NHCOR⁴, -(CH₂)_{d}-NR⁴COR⁵, -(CH₂)_{d}-OR⁶ or -(CH₂)_{d}-CO₂R⁶;
Ring **A** represents a piperidinyl, piperazinyl, pyrrolidinyl or azetidinyl group;
Ring **B** represents a phenyl, pyridinyl or pyrimidinyl group (optionally substituted with one or more groups independently selected from halogen, CN, CONH₂, CF₃, OCF₃, R⁷, and-(CH₂)_{f}-OR⁸);
R², R³, R⁴, R⁵and R⁷ independently represent H, straight or branched C₁₋₆ alkyl, -(CH₂)_{c}-[C₃₋₈ cycloalkyl], phenyl, benzyl, pyridyl or pyrimidyl;
or R² and R³, or R⁴ and R⁵ together with the nitrogen atom to which they are attached independently represent a heterocycle of from 3 to 8 atoms;
R⁶ and R⁸ independently represent H, straight or branched C₁₋₆ alkyl, -(CH₂)_{c}-[C₃₋₈ cycloalkyl], -(CH₂)ₑ-NR⁴R⁵, -(CH₂)ₑ-OR⁴, phenyl, benzyl, pyridyl or pyrimidyl;
n=0, 1 or 2;
a , c, d and f are each independently selected from 0, 1, 2 and 3;
b and e are each independently selected from 2 and 3,
for the manufacture of a medicament for the treatment of premature ejaculation.

In the above definitions, halogen means fluoro, chloro, bromo or iodo. Alkyl groups containing the requisite number of carbon atoms, except where indicated, can be unbranched or branched chain. Examples include methyl, ethyl, n-propyl, i-propyl, n-butyl, i-butyl, sec-butyl and t-butyl. Examples of cycloalkyl include cyclopropyl, cyclobutyl, cyclopentyl, cyclohexyl and cycloheptyl.

Heterocycles included within the definition of "heterocycle" are pyrrolyl, imidazolyl, triazolyl, thienyl, furyl, thiazolyl, oxazolyl, thiadiazolyl, oxadiazolyl, pyridinyl, pyrimidinyl, pyridazinyl, pyrazinyl, indolyl, isoindolyl, quinolinyl, isoquinolinyl, benzimidazolyl, quinazolinyl, phthalazinyl, benzoxazolyl and quinoxalinyl, together with partially or fully saturated versions thereof as well as azetidinyl, pyrrolidinyl, piperidinyl, piperazinyl, homopiperazinyl and morpholinyl.

Preferred groups of compounds are those in which:
(i) W is NR¹;
(ii) W is O;
(iii) R¹ is C₁-C₆ alkyl, and more preferably methyl, i-propyl or n-butyl;
(iv) R¹ is H;
(v) R¹ is -COR²
(vi) R¹ is -SO₂R²
(vii) R¹ is -CO-(CH₂)ₐ-NR²R³;
(viii) R² is C₁-C₆ alkyl, and more preferably methyl, i-propyl or t-butyl;
(ix) R² is -(CH₂)_{c}-[C₃-C₈ cycloalkyl], preferably cyclopropyl;
(x) R³ is C₁-C₆ alkyl, and more preferably methyl, i-propyl or n-butyl;
(xi) X is H;
(xii) Y is in the 4-position of the aromatic ring to which it is attached;
(xiii) Y is halogen, preferably chloro;
(xiv) ring **A** is linked to ring **B** via a nitrogen atom
(xv) ring **A** is piperidinyl;
(xvi) ring **A** is piperazinyl;
(xvii) ring **B** is pyridinyl, preferably 2-pyridinyl;
(xviii) ring **B** is pyrimidinyl, preferably 2-pyrimidinyl;
(xix) ring **B** is phenyl;
(xx) ring **B** is unsubstituted;
(xxi) n is 1;
(xxii) n is 2.

Preferred compounds according to the present invention are:
1-(3,4,5,6-Tetrahydro-2H- [1,2']bipyridinyl-4-yl)-5,6-dihydro-4H-2,3,5,10b-tetraaza-benzo[e]azulene;
5-Methyl-1-(3,4,5,6-tetrahydro-2H-[1,2']bipyridinyl-4-yl)-5,6-dihydro-4H-2,3,5,10b-tetraaza-benzo[e]azulene;
1-[1-(3,4,5,6-Tetrahydro-2H-[1,2']bipyridinyl-4-yl)-4H,6H-2,3,5,10b-tetraaza-benzo[e]azulen-5-yl]-ethanone;
8-Chloro-1-(3,4,5,6-tetrahydro-2H-[1,2']bipyridinyl-4-yl)-5,6-dihydro-4H-2,3,5,10b-tetraaza-benzo[e]azulene;
8-Chloro-5-methyl-1-(3,4,5,6-tetrahydro-2H-[1,2']bipyridinyl-4-yl)-5,6-dihydro-4H-2,3,5, 10b-tetraaza-benzo[e]azulene trihydrochloride;
8-Chloro-5-isopropyl-1-(3,4,5,6-tetrahydro-2H-[1,2']bipyridinyl-4-yl)-5,6-dihydro-4H-2,3,5,10b-tetraaza-benzo[e]azulene trihydrochloride;
8-Chloro-1-(3,4,5,6-tetrahydro-2H-[1,2']bipyridinyl-4-yl)-5-(tetrahydro-pyran-4-yl)-5,6-dihydro-4H-2,3,5, 10b-tetraaza-benzo[e]azulene;
1-[8-Chloro-1-(3,4,5,6-tetrahydro-2H-[1,2']bipyridinyl-4-yl)-4H,6H-2,3,5,10b-tetraaza-benzo[e]azulen-5-yl]-ethanone dihydrochloride;
[8-Chloro-1-(3,4,5,6-tetrahydro-2H-[1,2']bipyridinyl-4-yl)-4H,6H-2,3,5,1 10b-tetraaza-benzo[e]azulen-5-yl]-cyciopropyl-methanone dihydrochloride;
1-[8-Chloro-1-(3,4,5,6-tetrahydro-2H-[1,2']bipyridinyl-4-yl)-4H,6H-2,3,5,1 10b-tetraaza-benzo[e]azulen-5-yl]-2,2-dimethyl-propan-1-one dihydrochloride;
8-Chloro-5-methanesulfonyl-1-(3,4,5,6-tetrahydro-2H-[1,2']bipyridinyl-4-yl)-5,6-dihydro-4H-2,3,5,10b-tetraaza-benzo[e]azulene;
8-Chloro-1-(1-pyrimidin-2-yl-piperidin-4-yl)-5,6-dihydro-4H-2,3,5,1 10b-tetraaza-benzo[e]azulene;
8-Chloro-5-methyl-1-(1-pyrimidin-2-yl-piperidin-4-yl)-5,6-dihydro-4H-2,3,5,10b-tetraaza-benzo[e]azulene;
8-Chloro-5-isopropyl-1-(1-pyrimidin-2-yl-piperidin-4-yl)-5,6-dihydro-4H-2,3,5,1 10b-tetraaza-benzo[e]azulene;
8-Chloro-5-methanesulfonyl-1-(1-pyrimidin-2-yl-piperidin-4-yl)-5,6-dihydro-4H-2,3,5,10b-tetraaza-benzo[e]azulene;
[8-Chloro-1- (1-pyrimidin-2-yl-piperidin-4-yl)-4H, 6H-2,3,5,10b-tetraaza-benzo[e]azulen-5-y!]-cyclopropyl-methanone;
1-[8-Chloro-1-(1-pyrimidin-2-yl-piperidin-4-yl)-4H,6H-2,3,5,10b-tetraaza-benzo[e]azulen-5-yl]-2,2-dimethyl-propan-1-one;
1-[8-Ch)oro-1-(1-pyrimidin-2-yl-piperidin-4-yl)-4H,6H-2,3,5,10b-tetraaza-benzo[e]azulen-5-yl]-ethanone;
8-Chloro-1-(6'-trifluoromethyl-3,4,5,6-tetrahydro-2H-[1,2']bipyridinyl-4-yl)-4H,6H-5-oxa-2,3,10b-triaza-benzo[e]azulene;
4-(8-Chloro-4H,6H-5-oxa-2,3,10b-triaza-benzo[e]azulen-1-yl)-3,4,5,6-tetrahydro-2H-[1,2']bipyridinyl-6'-carbonitrile;
4-(8-Chloro-4H,6H-5-oxa-2,3,10b-triaza-benzo[e]azulen-1-yl)-3,4,5,6-tetrahydro-2H-[1,2']bipyridinyl-6'-carboxylic acid amide;
13-Chloro-3-(3,4,5,6-tetrahydro-2H-[1,2']bipyridinyl-4-yl)-2,4,5,8-tetraaza-tricyclo[9.4.0.0*2,6*]pentadeca-1(11),3,5,12,14-pentaene;
1-[13-Chloro-3-(3,4,5,6-tetrahydro-2H-[1,2']bipyridinyl-4-yl)-2,4,5,8-tetraazatricyclo[9.4.0.0*2,6*]pentadeca-1(11),3,5,12,14-pentaen-8-yl]-ethanone;
13-Chloro-8-methyl-3-(3,4,5,6-tetrahydro-2H-[1,2']bipyridinyl-4-yl)-2,4,5,8-tetraazatricyclo[9.4.0.0*2,6*]pentadeca-1(11),3,5,12,14-pentaene;
3-(1-Pyrimidin-2-yl-piperidin-4-yl)-8-oxa-2,4,5-triazatricyclo[9.4.0.0*2,6*]pentadeca-1(11),3,5,12,14-pentaene;
8-Chloro-1-(1-pyrimidin-2-yl-piperidin-4-yl)-4H,6H-5-oxa-2,3,10b-triazabenzo[e]azulene;
13-Chloro-3-(3,4,5,6-tetrahydro-2H-[1,2']bipyridinyl-4-yl)-8-oxa-2,4,5-triaza-tricyclo[9.4.0.0*2,6*]pentadeca-1(11),3,5,12,14-pentaene;
3-(3,4,5,6-Tetrahydro-2H-[1,2']bipyridinyl-4-yl)-8-oxa-2,4,5-triaza-tricyclo[9.4.0.0*2,6*]pentadeca-1(11),3,5,12,14-pentaene dihydrochloride;
8-Chloro-1-(3,4,5,6-tetrahydro-2H-[1,2']bipyridinyl-4-yl)-4H,6H-5-oxa-2,3,10b-triaza-benzo[e]azulene;
7-Chloro-1-(3,4,5,6-tetrahydro-2H-[1,2']bipyridinyl-4-yl)-4H,6H-5-oxa-2,3,10b-triaza-benzo[e]azulene dihydrochloride;
1-(3,4,5,6-Tetrahydro-2H-[1,2']bipyridinyl-4-yl)-4H,6H-5-oxa-2,3,10b-triaza-benzo[e]azulene;
8-Methoxy-1-(3,4,5,6-tetrahydro-2H-[1,2']bipyridinyl-4-yl)-4H,6H-5-oxa-2,3,10b-triaza-benzo[e]azulene dihydrochloride;
8-Fluoro-1-(3,4,5,6-tetrahydro-2H-[1,2']bipyridinyl-4-yl)-4H,6H-5-oxa-2,3,10b-triaza-benzo[e]azulene;
8,9-Difluoro-1-(3,4,5,6-tetrahydro-2H-[1,2']bipyridinyl-4-yl)-4H,6H-5-oxa-2,3,10b-triaza-benzo[e]azulene dihydrochloride;
9-Chloro-1-(3,4,5,6-tetrahydro-2H-[1,2']bipyridinyl-4-yl)-4H,6H-5-oxa-2,3, 10b-triaza-benzo[e]azulene dihydrochloride;
1-(3,4,5,6-Tetrahydro-2H-[1,2']bipyridinyl-4-yl)-8-trifluoromethoxy-4H,6H-5-oxa-2,3,10b-triaza-benzo[e]azulene;
8-Methyl-1-(3,4,5,6-tetrahydro-2H-[1,2']bipyridinyl-4-yl)-4H,6H-5-oxa-2,3,10b-triaza-benzo[e]azulene; and
1-[8-Chloro-1-(3,4,5,6-tetrahydro-2H-[1,2']bipyridinyl-4-yl)-4H,6H-2,3,5,10b-tetraaza-benzo[e]azulen-5-yl]-2-dimethylamino-ethanone; or pharmaceutically acceptable derivatives thereof.

A process for the production of a compound of formula (I) comprises:
a) reacting a compound of formula **(II)** with an acid catalyst 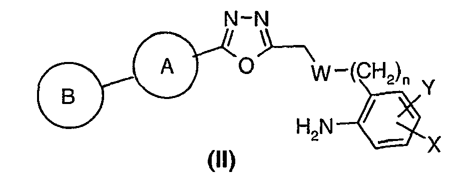 wherein rings A and B, and groups W, X, Y and n are as defined above.
b) reacting a compound of formula **(III)** 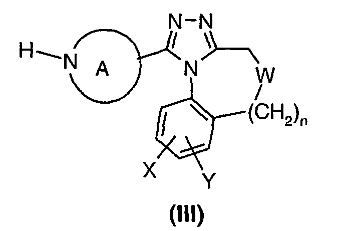 with a compound of formula **(IV)** 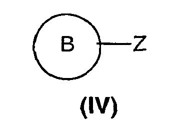 wherein rings A and B, and groups W, X, Y and n are as defined above, and Z represents a leaving group such as halogen.
c) when W in compound **(I)** represents NR¹, reacting a compound of formula **(V)** 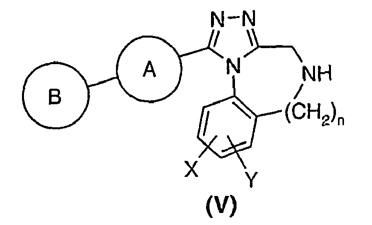 with a compound of formula **(VI)** 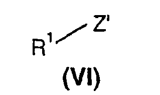 wherein rings A and B, and groups R¹, X, Y and n are as defined above, and Z' represents a leaving group such as halogen.
**d)** When W in compound (I) represents NR¹, reacting a compound of formula **(V)** 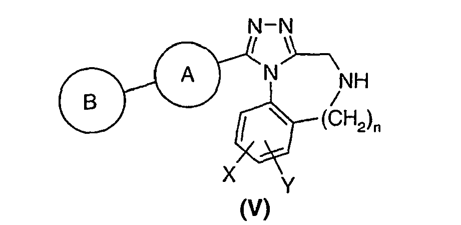 with a compound of formula **(VII)** 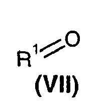 wherein rings A and B, and groups R¹, X, Y and n are as defined above.

Unless otherwise provided herein:
WSCDI means 1-(3-dimethylaminopropyl)-3-ethylcarbodiimide hydrochloride;
DCC means N,N'-dicyclohexylcarbodiimide;
HOAT means 1-hydroxy-7-azabenzotriazole;
HOBT means 1-hydroxybenzotriazole hydrate;
PyBOP^{®} means Benzotriazol-1-yloxytris(pyrrolidino)phosphoniumhexa fluorophosphate;
PyBrOP^{®} means bromo-tris-pyrrolidino-phosphoniumhexafluoro phosphate;
HBTU means O-Benzotriazol-1-yl-N,N,N',N'-tetramethyluronium hexafluorophosphate.
Mukaiyama's reagent means 2-chloro-1-methylpyridinium iodide;
KHMDS means potassium bis(trimethylsilyl)amide;
Hünig's base means N-ethyldiisopropylamine;
Et₃N means triethylamine;
NMM means N-methylmorpholine;
HMDS means hexamethyldisilazane
BINAP means 2,2'-bis(diphenylphosphino)-1,1'-binaphthyl;
Dba means dibenzylideneacetone;
Boc means *tert*-butoxycarbonyl;
CBz means benzyloxycarbonyl;
p-TSA means *p*-toluenesulphonic acid
TBAF means tetra-butyl ammonium fluoride
MeOH means methanol, EtOH means ethanol, and EtOAc means ethyl acetate;
THF means tetrahydrofuran, DMSO means dimethyl sulphoxide, and DCM means dichloromethane, DMF means N,N-dimethylformamide, NMP means N-methyl-2-pyrrolidinone;
AcOH means acetic acid, TFA means trifluoroacetic acid.

The following schemes illustrate the preparation of compounds of the formula (I), throughout which Rings A and B, and groups W, X, Y, and n are as defined above unless otherwise stated. (I') represents (I) when W is NR¹.

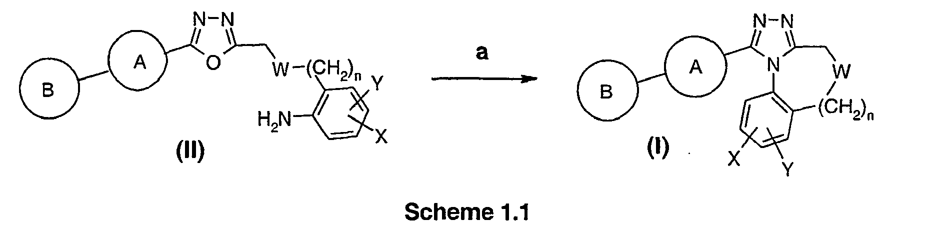

**Step (a)**: Oxadiazole **(II)** is reacted with an acid catalyst to give the compound of formula **(V)**. Typically the reaction is carried out by heating the starting materials to elevated temperatures, such as 100-150°C, for 1 to 48 hours with a suitable acidic catalyst such as p-TSA, or Lewis acid catalyst such as magnesium chloride, optionally using a high boiling solvent such as xylene. Preferred conditions are:
Amine **(II)** and cat. P-TSA, in xylene at 140°C for 48 hrs.

When W = NR¹, then: 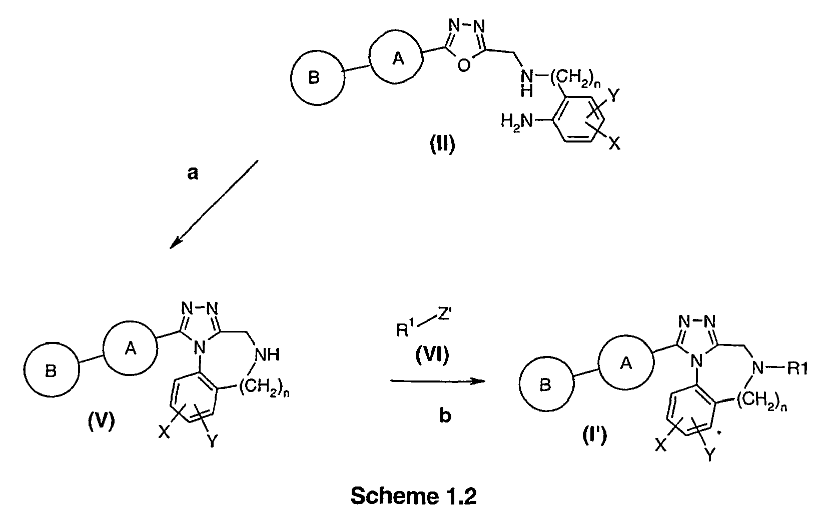 Z' is OH or halo, typically Cl

Compounds suitable for use as compound **(VI)** are commercially available or are known in the literature.

**Step (b):** The reaction of amine **(V)** with compound **(VI)** can be carried out by standard methods.

When R¹ = COR², CO₂R², CO-(CH₂)_{b}-NR³R⁴, SO₂R² then, typically, the coupling may be undertaken by using:
(i) an acyl/sulphonyl/ chloride **(VI)** + amine **(V)** with an excess of acid acceptor, in a suitable solvent; or
(ii) an acid **(VI)** with a conventional coupling agent + amine **(V),** optionally in the presence of a catalyst, with an excess of acid acceptor in a suitable solvent; and
(iii) when R¹ represents an Aryl group, an aryl halide **(VI)** + amine **(V),** optionally in the presence of a catalyst, with an excess of acid acceptor in a suitable solvent.

Typically the conditions are as follows:

### Acylation/Sulphonylation, Z=Cl

(i) An excess of acyl/sulphonyl chloride **(VI)** (generated in-situ), 1 eq. of amine **(V)**, optionally with an excess of 3° amine such as Et₃N, Hünig's base or NMM, in DCM or THF, without heating for 1 to 24 hrs.
   The preferred conditions are:
   Amine **(V)**, 1.5 eq. acid/sulphonyl chloride **(VI)**, 1.5 eq. NMM in DCM at rt. for 16 hours.

### Amide Bond Formation, Z=OH

(ii) Excess acid **(VI),** WSCDI /DCC and HOBT/HOAT, 1 eq. of amine **(V),** with an excess of NMM, Et₃N, Hünig's base in THF, DCM or EtOAc, at rt. for 4 to 48 hrs; or
   excess acid **(VI),** PYBOP®/PyBrOP®/Mukaiyama's reagent, 1 eq. of amine **(V),** with an excess of NMM, Et₃N, Hünig's base in THF, DCM or EtOAc, at rt. for 4 to 24 hrs.

### Arylation (R¹ = Aryl, heteroaryl), Z = halo

(iii) Arylation of compound **(V)** can be carried out by a palladium catalysed cross-coupling reaction using a suitable base (*t*-BuONa), a catalytic amount of suitable additive such as 2,2'-bis(diphenylphosphino)-1,1'-binaphthyl and a suitable palladium catalyst in toluene at elevated temp for 1 to 24 hours under an inert atmosphere, to give compound **(I').** Alternatively compound **(I')** can be prepared by reaction of the amine **(I)** with compound **(VI)** by heating at elevated temperature, such as 50°C-140°C, in a suitable solvent such as DMF, NMP or 1,4-dioxan for about 1-48 hrs with a base such as potassium carbonate, sodium hydrogen carbonate or Hünig's base.

Preferred conditions are:
1-2.5 eq. halide **(VI),** 1-2 eq. potassium carbonate in N,N-dimethylformamide at 50 °C for 4-18 hours; or
1-2.5 eq. halide **(VI),** 2-3 eq. Hünig's base, in 1,4-dioxan or NMP at reflux for 18-48 hrs; or
1 eq. Halide **(VI),** 3.5 eq. NaOt-Bu, 0.08eq BINAP, 0.4 eq. Pd(dba)₂, in toluene for 8 hrs at 70°C.

Alternatively, compounds **(I')** may be prepared by the route shown below in **scheme 1.3.**

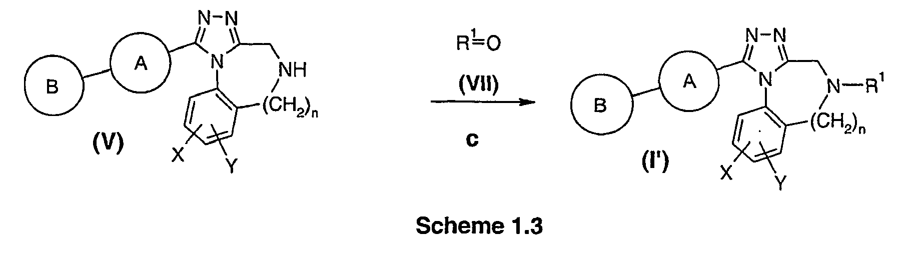

Compounds suitable for use as compound **(VII)** are commercially available or are known in the literature.

**Step (c)**: Amine **(V)** is reacted with an excess of aldehyde/ketone **(VII)** in the presence of a reducing agent, such as sodium triacetoxy borohydride or sodium cyanoborohydride, to give the compound of formula **(I').** This reaction may be carried out by:
stirring the starting materials at temperatures such as 20°C-80°C for 1 to 48 hours in a suitable solvent such as dichloromethane, or
heating amine **(V)** with excess compound **(VII)** with a suitable Lewis acid catalyst such titanium tetrachloride or titanium tetraisopropoxide at temperatures such as 50°C-100°C in a suitable solvent such as dichloroethane or ethanol for 1-18 hours, followed by reduction of the intermediate imine/iminium species with a suitable reducing agent, such as sodium borohydride, or hydrogenolysis over a suitable catalyst, such as platinum oxide or palladium on carbon.

Preferred conditions are:
Amine **(V),** 1.5 eq. Aldehyde/ketone **(VII),** 2.0 eq. sodium triacetoxy borohydride in dichloromethane at room temperature for 2 hours.

When ring B is linked to ring A via an N atom, and W represents O or S then:

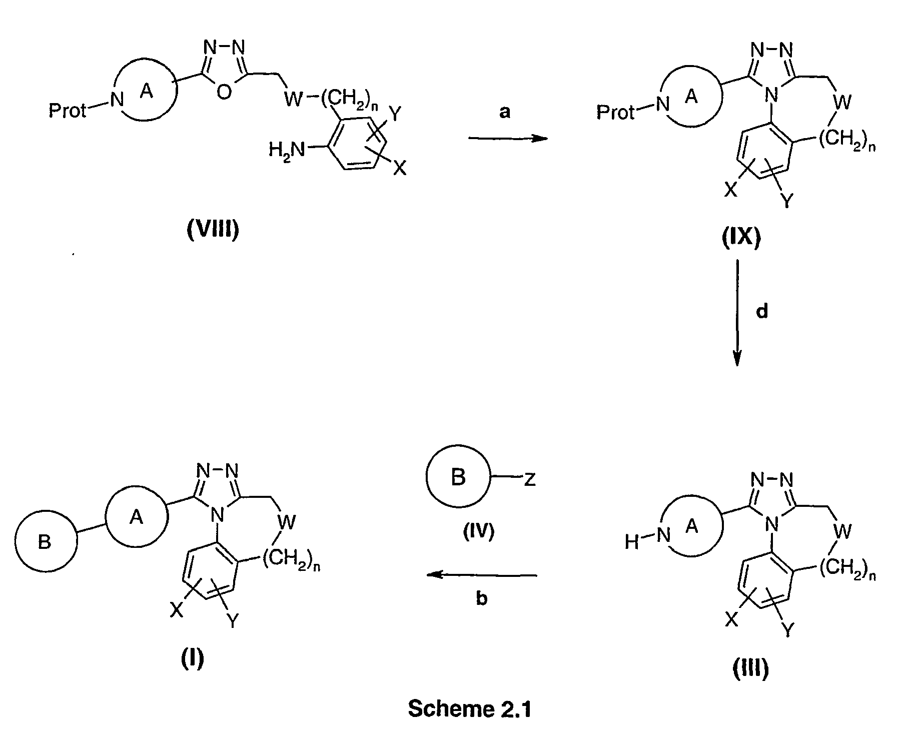

Prot represents a suitable protecting group for nitrogen, for example Boc, CBz or Allyl carbamate. Standard methodology for nitrogen protecting groups is used, such as that found in textbooks (e.g. "Protecting Groups in Organic Synthesis" by T.W. Greene and P. Wutz). Z represents a leaving group such as halogen.

Compounds suitable for use as compound **(IV)** are commercially available or are known in the literature.

Arylation of compound **(III)** can be carried out as described in **Step (b)** above.

Preferred conditions are:
1-2.5 eq. halide **(IV),** 1-2 eq. potassium carbonate in N,N-dimethylformamide at 50 °C for 4-18 hours; or
1-2.5 eq. halide **(IV),** 2-3 eq. Hünig's base, in 1,4-dioxan or NMP at reflux for 18-48 hrs; or
1 eq. halide **(IV),** 3.5 eq. NaOt-Bu, 0.08eq BINAP, 0.4 eq. Pd(dba)₂, in toluene for 8 hrs at 70°C.

**Step (d):** Deprotection of compound **(IX)** is undertaken using standard methodology, as described in "Protecting Groups in Organic Synthesis" by T.W. Greene and P. Wutz".

When Prot is Boc, the preferred methods are:
hydrogen chloride in a suitable solvent such as 1,4-dioxane at room temperature for 1-16 hours; or
a solution of trifluoroacetic acid in dichloromethane for 1-2 hours.

When Prot is CBz, the preferred method is hydrogenolysis using a suitable palladium catalyst in a solvent such as ethanol.

When Prot is an allyl carbamate, preferred conditions are thiobenzoic acid and a suitable palladium catalyst such as Pd₂(Dba)₃ with a suitable phosphine additive such as 1,4-bis(diphenylphosphino)butane in tetrahydrofuran for 20 minutes.

When ring B is linked to ring A via an N atom, and W represents NR¹ then:

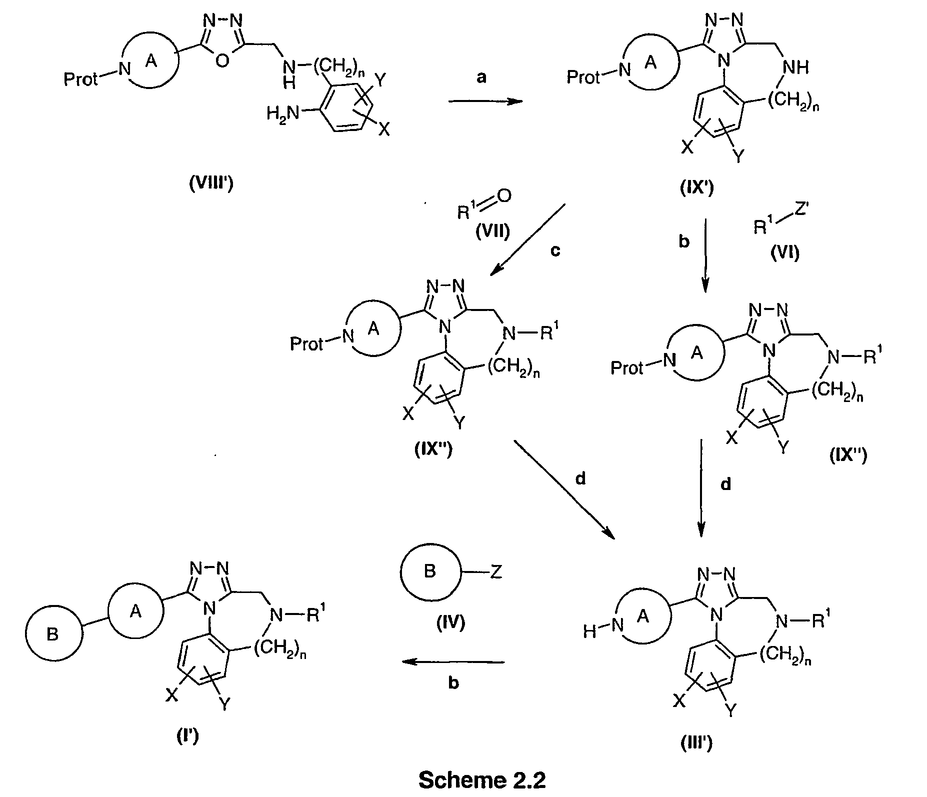

Prot represents a suitable protecting group for nitrogen, for example Boc, CBz or Allyl carbamate. Standard methodology for nitrogen protecting groups is used, such as that found in textbooks, (e.g. "Protecting Groups in Organic Synthesis" by T.W. Greene and P. Wutz).

Z represents halo (typically CI). Z' represents a leaving group (typically Cl or OH).

Compounds suitable for use as compound **(IV)** are commercially available or are known in the literature.

Compound **(IX")** typically can be prepared from compound **(IX')** using the methodology described in **Step (b)** and **Step (c)** above.

Compound **(III')** typically can be prepared from compound **(IX")** using the methodology described in **Step (d)** above.

Compounds **(I')** typically can be prepared by arylation of compounds **(III')** using the methodology described in **Step (b)** above.

Compounds suitable for use as compounds **(II)** and **(VIII)** are known in the literature or can be prepared as shown in schemes 3.1 and 3.2 below.

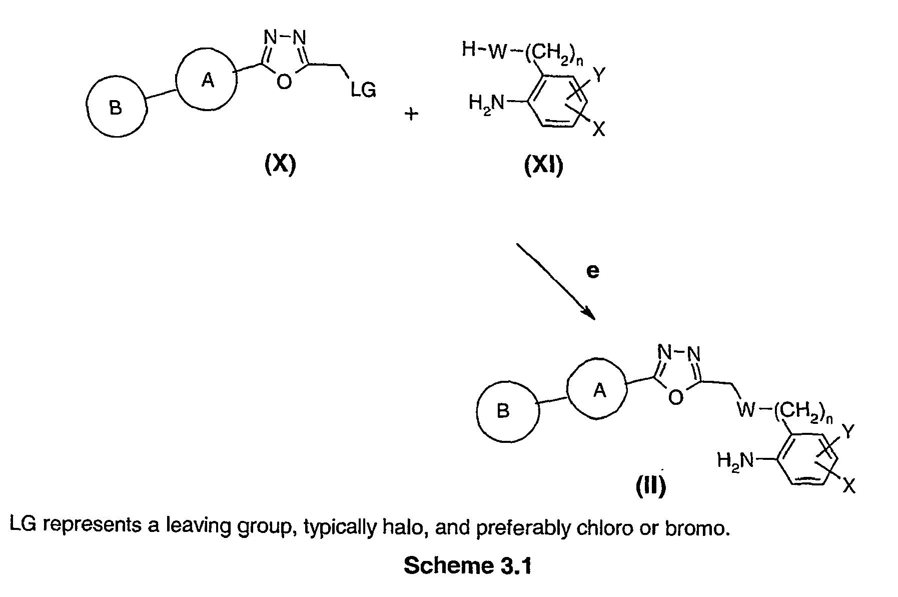

When rings A and B are linked through an N atom then:

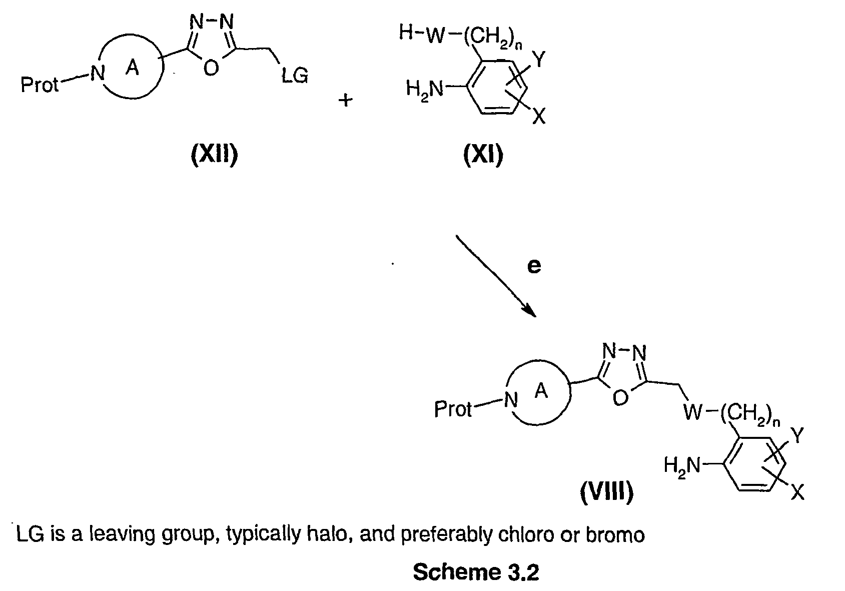

Compounds suitable for use as compounds **(XI)** are known in the literature or can be prepared using standard methodology: for example, reduction of benzoic acids (see preparation 7 below) or benzonitriles (see preparation 10 below).

When W represents NR¹:

**Step (e):** Compound **(X)/(XII)** is reacted with an excess of compound **(XI)** to give compound **(II)/(VIII)** respectively, optionally in the presence of an excess of base, such as triethylamine, Hunig's base or potassium carbonate as proton acceptor, in a suitable high boiling solvent such as THF, Toluene or DMF at temperatures from 50°C to 100°C for 1 to 48 hours.

Preferred conditions are:
2.5 eq. of compound **(XI)** in THF at 50°C for 48 hours.

When W represents O or S:

**Step (e)**: Compound **(X)/(XII)** is reacted with an excess of compound **(XI)** in the presence of a base such as sodium hydride, potassium hexamethyldisilazide, ⁿbutyl lithium or isopropyl magnesium chloride, in a suitable solvent such as THF, Toluene or NMP at temperatures from 0°C to 50°C for 1 to 24 hours, to give compound **(II)/(VIII)** respectively.

Preferred conditions are:
3 eq. of compound (XI) and 2.5 eq. of NaH in THF at 20°C for 2 hours.

Compounds suitable for use as compounds (X) and (XII) are known in the literature or can be prepared as shown in scheme 4.1 and 4.2.

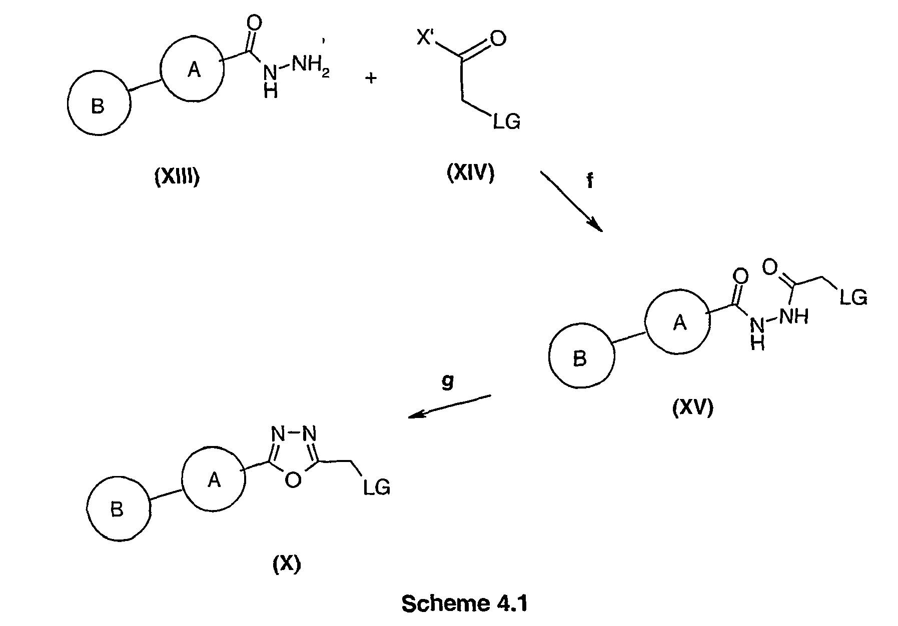

X' represents OH or halo, and preferably represents Cl. LG represents a leaving group, typically halo, and preferably chloro or bromo

When rings A and B are linked through an N atom then:

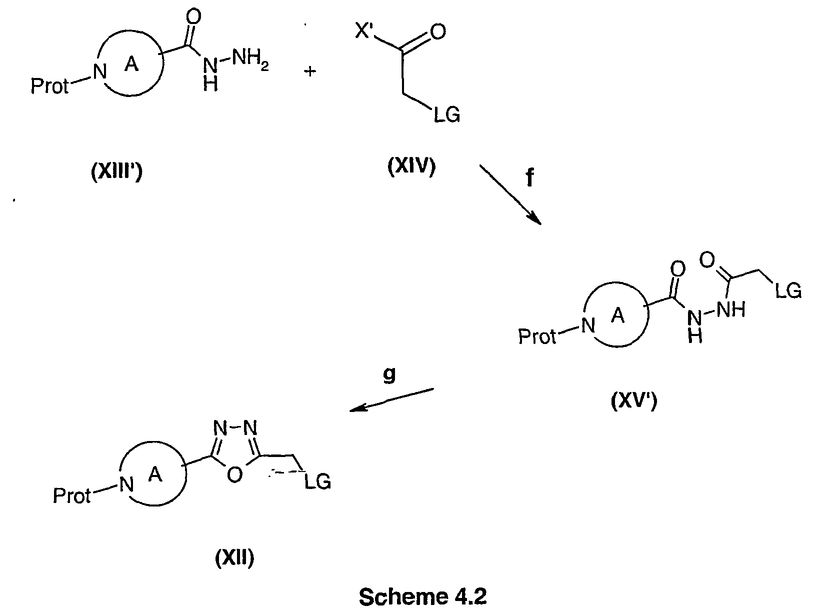

X' represents OH or halo, and preferably represents Cl. LG is a leaving group, typically halo, and preferably chloro or bromo

Compound **(XIV)** is either commercially available or is known in the literature.

**Step (f):** The reaction of hydrazide **(XIII/XIII')** with compound **(XIV)** can be carried out by standard methods.

Coupling may be undertaken by using either:
(i) an acyl chloride **(XIV)** + hydrazide **(XIII/XIII')** with an excess of acid acceptor in a suitable solvent; or
(ii) acid **(XIV)** with a conventional coupling agent + hydrazide **(XIII/XIII'),** optionally in the presence of a catalyst, with an excess of acid acceptor in a suitable solvent.

Typically the conditions are as follows:
(i) acid chloride **(XIV)** (generated in-situ), an excess of hydrazide **(XIII/XIII'),** optionally with an excess of 3° amine such as Et₃N, HOnig's base or NMM, in DCM or THF, without heating for 1 to 24 hrs; or
(ii) acid **(XIV),** WSCDI /DCC and HOBT /HOAT, an excess of hydrazide **(XIII/XIII'),** with an excess of NMM, Et₃N, Hünig's base in THF, DCM or EtOAc, at rt. for 4 to 48 hrs; or
(ii) acid **(XIV),** PYBOP^{®}/PyBrOP^{®}/Mukaiyama's reagent, an excess of hydrazide **(XIII/XIII'),** with an excess of NMM, Et₃N, Hünig's base in THF, DCM or EtOAc, at rt. for 4 to 24 hrs.

The preferred conditions are:
Hydrazide **(XIII/XIII'),** 1.5 eq. chloro acetyl chloride **(XIV),** 1.5 eq. NMM in DCM at rt. for 16 hours.

**Step (g):** Cyclisation of compound **(XV/XV')** is carried out under suitable dehydrating conditions, at elevated temperatures for up to 18 hours.
Typically, dehydrating agents such as polyphosphoric acid, phosphorous oxychloride, triflic anhydride are used at temperatures from 20 to 120°C for 5 minutes to 12 hours. Optionally, the reaction can be carried out in the presence of a base such as pyridine and suitable solvents such as dichloromethane and acetonitrile. Alternatively, the oxadiazole **(XII/X)** may be prepared according to the method of Rigo et. al. Synth. Commun. 16(13), 1665, 1986.

Preferred conditions are:
Phosphorous oxychloride at 100°C for 8 hours, or 2.5 eq. triflic anhydride, 5 eq. pyridine in dichloromethane at 20°C for 3 hours.

Compounds suitable for use as compounds **(XIII/XIII')** are known in the literature or can be prepared as shown in scheme 5.1 and 5.2.

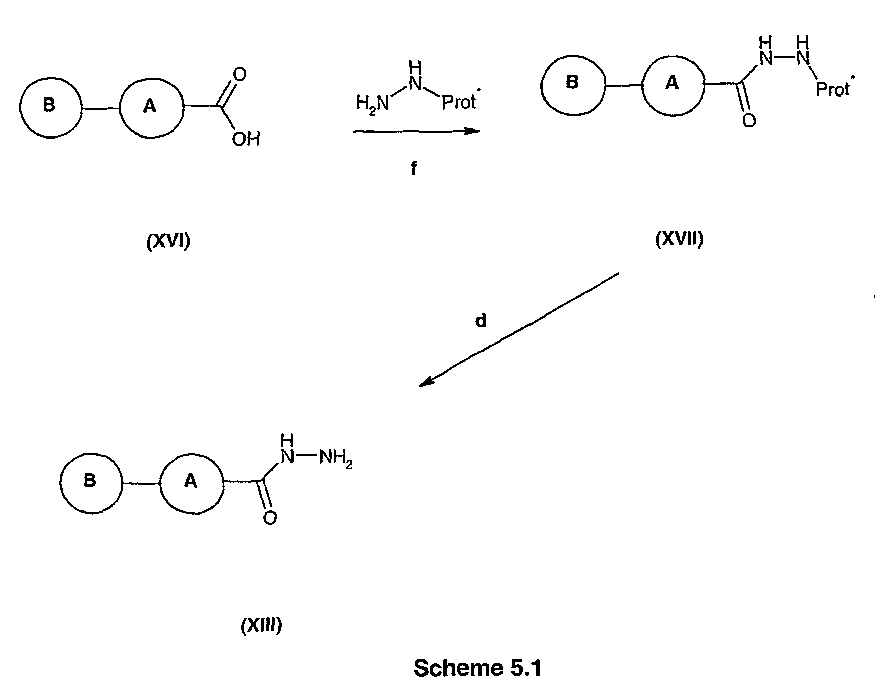

When rings A and B are linked through an N atom then:

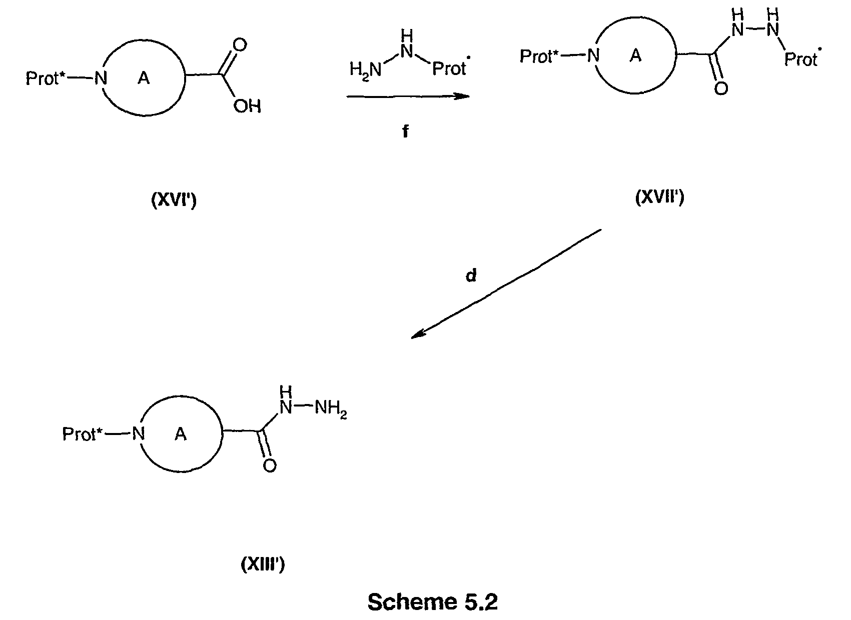

Compounds **(XVI)/(XVI')** and protected hydrazine are either commercially available or are known in standard methodology such as the hydrolysis of the corresponding ester.

Carboxylic acid **(XVI)/(XVI')** and protected hydrazine, where prot* is typically Boc, may be coupled to give compound **(XVII/XVII')** respectively, using the conditions described above for the preparation of **(XV/XV'),** and then prot* is removed using standard methodology as described in **Step (d)** as described above, to give **(XIII/XIII').**

Alternative routes to compound **(XIII/XIII')** are shown below in schemes 6.1 and 6.2:

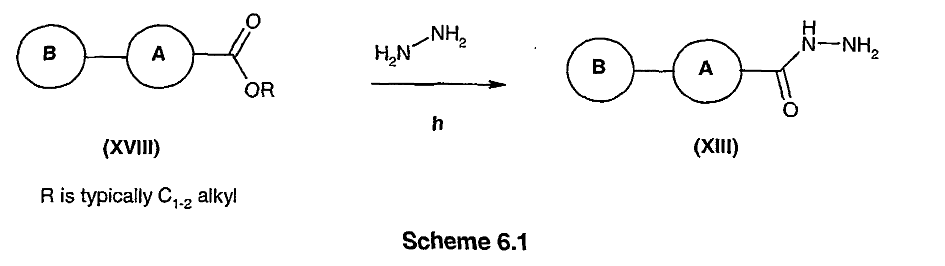

When rings A and B are linked through an N atom then:

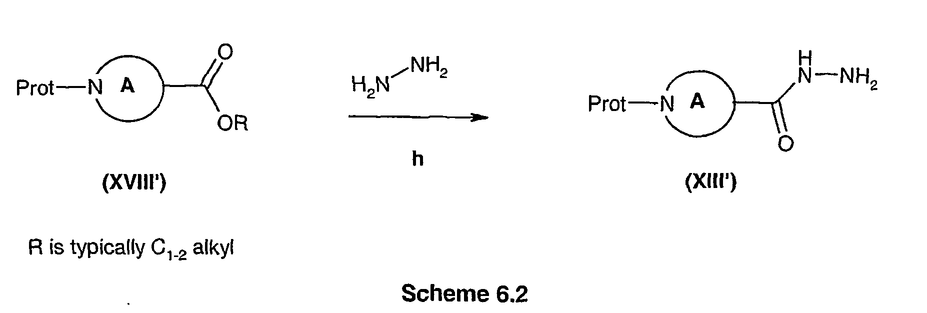

**Step (h):** The ester **(XVIII/XVIII')** may be reacted with hydrazine in a suitable solvent, such as methanol, at an elevated temperature to provide the hydrazide **(XVII/XVII').** Preferred conditions:
3 eq. hydrazine, in methanol, at reflux for 18 hrs.

It will be apparent to those skilled in the art that sensitive functional groups may need to be protected and deprotected during synthesis of a compound of formula **(I).** This may be achieved by conventional techniques, for example as described in "Protective Groups in Organic Synthesis" by T W Greene and P G M Wuts, John Wiley and Sons Inc, 1991.

In accordance with the present invention there is further provided an intermediate of formula **(II):** 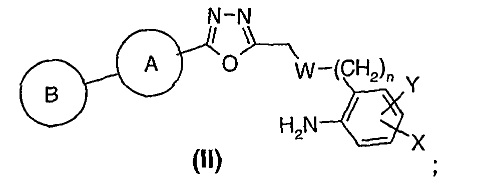 an intermediate of formula **(XV):** 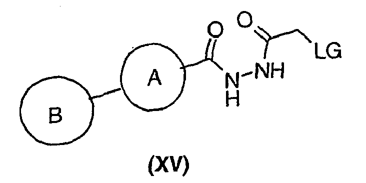 an intermediate of formula **(X):** 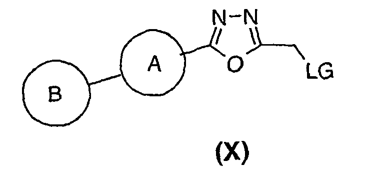 wherein X, Y, W, rings **A** and **B,** LG and n are as defined above.

A suitable vasopressin V1a receptor antagonist is, for example, Compound 1: (4-[4-Benzyl-5-(4-methoxy-piperidin-1-ylmethyl)-4*H*-[1,2,4Jtriazol-3-yl]-3,4,5,6-tetrahydro-2*H-*[1,2']bipyridinyl), which is Example 26 in WO 04/037809.

Further examples of vasopressin V1a receptor antagonists for use with the invention are disclosed in WO 04/037809 and PCT/IB 2004/000432. In particular 8-chloro-5-Methyl-1-(3,4,5,6-tetrahydro-2H-[1,2']bipyridinyl-4-yl)-5,6-dihydro-4H-2,3,5,10b-tetraazo-benzo[e]azulene, or a pharmaceutically acceptable salt or solvate thereof, is preferred. Another preferred V1a antagonist for use with the invention is 8-Chloro-5-methyl-1-(1-pyrimidin-2-yl-piperidin-4-yl)-5,6-dihydro-4H-2,3,5,10b-tetraaza-benzo[e]azulene, or a pharmaceutically acceptable salt or solvate thereof.

Further examples of suitable vasopressin V1 a receptor antagonists are disclosed in US 6,090,818; EP0873309; WO 98/25901; WO 02/083685; JP 2000-63363; and WO 02/32864.

Examples of vasopressin V1a receptor antagonists for use with the invention are: SR49049 (Relcovaptan), atosiban (Tractocile®), conivaptan (YM-087) and OPC21268.

Additionally, the V1a receptor antagonists described in WO 01/58880 are suitable for use in the invention.

Yet a further aspect of the invention is a method of screening for compounds useful for treating ejaculatory disorders, preferably premature ejaculation or rapid ejaculation, comprising screening compounds for antagonist activity against vasopressin V1a receptors, and selecting compounds with an IC₅₀ of less than 100nM, preferably with an IC₅₀ of less than 10nM, even more preferably with an IC₅₀ of less than 5nM.

Preferably, the method of screening further comprises testing the compounds for antagonist activity against oxytocin receptor and/or vasopressin V2 receptor and/or vasopressin V1b receptor, and selecting a compound with the desired selectivity for the V1a receptor.

The invention relates to the use of a vasopressin V1 a receptor antagonist for the treatment of ejaculatory disorders, preferably premature ejaculation or rapid ejaculation, alone, or in combination with one or more other agents such as
- A selective serotonin reuptake inhibitor (SSRI), particularly SSRis having a rapid onset and a short duration of action. Suitable SSRIs for use in the present invention Include: sertraline, fluoxetine, fluvoxamine, paroxetine, citalopram, dapoxetine, 3-[(dimethylamino)methyl]-4-[4-(methylsulfanyl)phenoxy] benzenesulfonamide (Example 28, WO 0172687), 3-[(dimethylamino)methyl]-4-[3-methyl-4-(methylsulfanyl)phenoxy] benzenesulfonamide (Example 12, WO 0218333), N-methyl-N-({3-[3-methyf-4-(methylsulfanyl)phenoxy]-4-pyridinyi}methyl)amine (Example 38, WO 02/083643). Preferred SSHIs are 3-[(dimethylamino)methyf)-A-[4-(methylsulfanyl)phenoxy] benzenesulfonamide (Example 28, WO 0172687), 3-[(dimethylamino)methyl]-4-[3-methyl-4-(methylsulfanyl)phenoxy] benzenesulfonamide (Example 12, WO 0218333), *N-*methyl-*N*-({3-[3-methyl-4-(methylsulfanyl)phenoxy]-4-pyridinyl}methyl)amine (Example 38, WO 02/083643), paroxetine and dapoxetine. More preferred are 3-[(dimethylamino)methyl]-4-[4-(methylsulfanyl)phenoxy]benzenesulfonamide (Example 28, WO 0172687), 3-[(dimethylamino)methyl]-4-[3-methyl-4-(methylsulfanyl)phenoxy] benzenesulfonamide (Example 12, WO 0218333), *N-*methyl-*N*-({3-[3-methyl-4-(methylsulfanyl)phenoxy]-4-pyridinyl}methyl)amine (Example 38, WO 02/083643).
- A PDE inhibitor, more particularly a PDE 5 inhibitor (see below), the inhibitors preferably having an IC₅₀ against the respective enzyme of less than 100nM;
- A serotonin receptor antagonist or modulator, more particularly antagonists or modulators for 5HT1A, Including NAD-299 (robalzotan) and WAY-100635, and/or more particularly antagonists or modulators for 5HT3 receptors, including batanopride, granisetron, ondansetron, tropistron and MDL-73147EF;
- A serotonin receptor agonist or modulator, more particularly agonists or modulators for 5HT2C, 5HT1B and/or 5HT1D receptors, Including eletriptan (WO92/06973), naratriptan (GB 2208646), rizatriptan (EP 497512), almotriptan (WO94/02460), avitriptan, sumatriptan (GB 2162522), frovatriptan, ainiditan, zolmitriptan (WO91/18897), LY 334370, LY 306258, BMS-180048, BMS-181885;
- An α-adrenergic receptor antagonist (also known as α-adrenergic blockers, a-blockers or a-receptor blockers); suitable a1-adrenergic receptor antagonists include: phentolamine, prazosin, phentolamine mesylate, trazodone, alfuzosin, indoramin, naftopidil, tamsulosin, phenoxybenzamine, rauwolfa alkaloids, Recordati 15/2739, SNAP 1069, SNAP 5089, RS17053, SL 89.0591, doxazosin, terazosin and abanoquil; suitable α2-adrenergic receptor antagonists include dibenamine, tolazoline, trimazosin, efaroxan, yohimbine, idazoxan clonidine and dibenarnine; suitable non-selective a-adrenergic receptor antagonists Include dapiprazole; further a-adrenergic receptor antagonists are described in WO99/30697, US4,188,390, US4,026,894, US3,511,836, US4,315,007, US3,527,761, US3,997,666, US2,503,059, US 4,703,063, US 3,381,009, US 4,252,721 and US 2,599,000 each of which is Incorporated herein by reference;
- Oxytocin receptor antagonists, e.g. L-368 899 (The synthesis of L-368,899 is taught in Williams et al (1994) J. Med. Chem. 37, 565-571).
- PDE inhibitors
   Suitable cGMP PDE inhibitors, preferably PDE 5 inhibitors, for the use in combination with vasopressin V1a receptor antagonists according to the invention include:
   The PDE5 inhibitors mentioned In international Patent Application publication nos. WO03/000691; WO02/64590; WO02/28865; WO02/28859; WO02/38563; WO02/36593; WO02/28858; WO02/00657; WO02/00656; WO02/10166; WO02/00658; WO01/94347; WO01/94345; WO00/15639 and WO00/15228; and
   US Patents 6,143,746; 6,143,747 and 6,043,252;
   the pyrazolo [4,3-d]pyrimidin-7-ones disclosed in EP-A-0463756; the pyrazolo [4,3-d]pyrimidin-7-ones disclosed in EP-A-0526004; the pyrazolo [4,3-d]pyrimidin-7-ones disclosed in published international patent application WO 93/06104; the isomeric pyrazolo [3,4-d]pyrimidin-4-ones disclosed in published international patent application WO 93/07149; the quinazolin-4-ones disclosed in published international patent application WO 93/12095; the pyrido [3,2-d]pyrimidin-4-ones disclosed in published international patent application WO 94/05661; the purin-6-ones disclosed in published international patent application WO 94/00453; the pyrazolo [4,3-d]pyrimidin-7-ones disclosed in published international patent application WO 98/49166; the pyrazolo [4,3-d]pyrimidin-7-ones disclosed in published international patent application WO 99/54333; the pyrazolo [4,3-d]pyrimidin-4-ones disclosed In EP-A-0995751; the pyrazolo [4,3-d]pyrimidin-7-ones disclosed in published international patent application WO 00/24745; the pyrazolo [4,3-d]pyrimidin-4-ones disclosed in EP-A-0995750; the hexahydropyrazino [2',1':6,1]pyrido [3,4-*b*]indole-1,4-diones disclosed in published International application WO95/19978; the pyrazolo [4,3-d]pyrimidin-4-ones disclosed in WO00/27848; the imidazo[5,i-*f*][1,2,4]triazin-ones disclosed In EP-A-1092719 and in published International application WO 99/24433 and the bicyclic compounds disclosed in published international application WO 93/07124; the pyrazolo [4,3-d]pyrimidin-7-ones disclosed In published international application WO 01/27112; the pyrazolo [4,3-d]pyrimidin-7-ones disclosed In published international application WO 01/27113; the compounds disclosed in EP-A-1092718 and the compounds disclosed in EP-A-1092719; the tricyclic compounds disclosed in EP-A-1241170; the alkyl sulphone compounds disclosed in published international application WO 02/074774; the compounds disclosed in published international application WO 02/072586; the compounds disclosed In published international application WO 02/079203 and the compounds disclosed in WO 02/074312.

The contents of the published patent applications and journal articles and in particular the general formulae of the therapeutically active compounds of the claims and exemplified compounds therein are incorporated herein in their entirety by reference.

Preferred type V phosphodiesterase inhibitors (PDE5 inhibitors) for the use according to the present invention include:
5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil, e.g. as sold as Viagra®) also known as 1-[[3-(6,7-dihydro-1-mathyl-7-oxo-3-propyl-1H-pyrazolo[4,3-d]pyrimidin-5-yl)-4-ethoxyphenyl]sulphonyl]-4-methylpiperazine (see EP-A-0463756);
5-(2-ethoxy-5-morpholinoacetytphenyl)-1-methyl-3-n-propyl-1,6-dihydro-7H-pyrazolo[4,3-d]pyrimidin-7-one (see EP-A-0526004);
3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrlmidin-7-one (see WO98/49166);
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one (see WO99/54333);
(+)-3-ethyl-5-[5-(4-ethylpiperazin-1-ylsulphonyl)-2-(2-methoxy-1 (R)-methylethoxy)pyridin-3-yl]-2-methyl-2,6-dihydro-7H-pyrazolo[4,3-dJpyrimidin-7-one, also known as 3-ethyl-5-{5-[4-ethylpiperazin-1-ylsulphonyl]-2-([(1R)-2-methoxy-1-methylethyl]oxy)pyridin-3-yl}-2-methyl-2,6-dihydro-7H-pyrazoto[4,3-d] pyrimidin-7-one (see WO99/54333
5-[2-ethoxy-5-(4-ethylpiperazin-1-ylsuiphonyl)pydin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see WO 01/27113, Example 8);
5-[2-*iso*-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (see WO 01/27113, Example 15);
5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see WO 01/27113, Example 66);
5-(5-Acetyl-2-propoxy-3-pyridinyl)-3-ethyl-2-(1-isopropyl-3-azetidinyl)-2,6-dihydro-7*H-*pyrazolo[4,3-d]pyrimidin-7-one (see WO 01/27112, Example 124); 5-(5-Acetyl-2-butoxy-3-pyridinyl)-3-ethyl-2-(1-ethyl-3-azetidinyl)-2,6-dihydro-7*H-*pyrazolo[4,3-d]pyrimidin-7-one (see WO 01/27112, Example 132);
(6R,12aR)-2,3,6,7,12,12a-hexahydro-2-methyl-6-(3,4-methylenedioxyphenyl)pyrazino [2',1':6,1]pyrido[3,4-b]indole-1,4-dione (tadalafil, IC-351, Cialis®), i.e. the compound of examples 78 and 95 of published international application WO95/19978, as well as the compound of examples 1, 3, 7 and 8;
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil, LEVITRA ®) also known as 1-[[3-(3,4-dihydro-5-methyl-4-oxo-7-propylimidazo[5,1-f]-as-triazin-2-yl)-4-ethoxyphenyl]sulphonyl]-4-ethylplperazine, I.e. the compound of examples 20, 19, 337 and 336 of published international application WO99/24433;
the compound of example 11 of published international application WO93/07124 (EISAI);
compounds 3 and 14 from Rotella D P, J. Med. Chem., 2000, 43, 1257;
4-(4-chlorobenzyl)amlno-6,7,8-trimethoxyquinazoline;
   N-[[3-(4,7-dihydro-1-methyl-7-oxo-3-propyl-1H-pymzolo[4,3-d]-pyrimidin-5-yl)-4-propxyphenyl]sulfonyl]-1-methyl2-pyrrolidinepropanamide ["DA-8159" (Example 68 of WOOO/27848)]; and
7,8-dihydro-8-oxo-6-[2-propoxyphenyl]-1*H*-imldazo[4,5-*g*]quinazoline and 1-[3-[1-[(4-fluorophanyl)methyl]-7,8-dihydro-8-oxo-1*H*-imidazo[4,5-*g*]quinazolin-6-yl]-4-propoxyphenyl]carboxamide.

Still other type cGMP PDE5 inhibitors which may be useful in conjunction with the present invention include:4-bromo-5-(pyridylmethylamino)-6-[3-(4-chlorophenyl)-propoxy]-3(2H)pyridazinone; 1-[4-[(1,3-benzodioxol-5-ylmethyl)amiono]-6-chloro-2-quinozolinyl]-4-piperidine-carboxylic acid, monosodium salt; (+)-cis-5,6a,7,9,9,9a-hexahydro-2-[4-(tritluoromethyl)-phenylmethyl-5-methyl-cyclopent-4,5]imidazo[2,1-b]purin-4(3H)one; furazlocillin; cis-2-hexyl-5-methyl-3,4,5,6a,7,8,9,9a-octahydrocyclopent[4,5]-imidazo[2,1-b]purin-4-one; 3-acetyl-1-(2-chlorobenzyl)-2-propylindole-6-carboxylate; 3-acetyl-1-(2-chlorobenzyl)-2-propylindole-6-carboxylate; 4-bromo-5-(3-pyridylmethylamino)-6-(3-(4-chlorophenyl) propoxy)-3- (2H)pyridazinone; l-methyl-5(5-morpholinoacetyl-2-n-propoxyphenyl)-3-n-propyl-1,6-dihydro- 7H-pyrazolo(4,3-d)pyrimidin-7-one; 1-[4-[(1,3-benzodioxol-5-ylmethyl)amino]-6-chloro-2-quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Schering Plough); GF-196960 (Glaxo Wellcome); E-8010 and E-4010 (Eisal); Bay-38-3045 & 38-9456 (Bayer); FR229934 and FR226807 (Fujisawa); and Sch-51866.

More preferably the PDE5 inhibitor is selected from sildenatil, tadalafil, vardenafil, DA-8159 and 5-[2-ethoxy-5-(4-ethytplperazln-1-ylsulphonyl)pyrldln-3-yij-3-ethyl-2-[2-methoxyethyl]-2,6-dihydro-7H-pyrazolo[4,3-d]pyrimidin-7-one.

Most preferably the PDE5 inhibitor is sildenafil and pharmaceutically acceptable salts thereof. Sildenafil citrate is a preferred salt.

Preferably, the cGMP PDE5 inhibitors have an IC₅₀ at less than 100 nanomolar, more preferably, at less than 50 nanomolar, more preferably still at less than 10 nanomolar.

The suitability of any particular cGMP PDE5 inhibitor can be readily determined by evaluation of its potency and selectivity using literature methods followed by evaluation of its toxicity, absorption, metabolism, pharmacokinetics, etc in accordance with standard pharmaceutical practice. Preferably the PDE5 inhibitors used in the pharmaceutical combinations are selective for the PDE5 enzyme. Preferably they have a selectivity of PDE5 over PDE3 of greater than 100, more preferably greater than 300. More preferably, the PDE5 inhibitor has a selectivity over both PDE3 and PDE4 of greater than 100, more preferably, greater than 300. Selectivity ratios may be determined readily by the skilled person. IC₅₀ values for the PDE3 and PDE4 enzyme may be determined using established literature methodology, see S.A. Ballard of al, J. Urology (1998) 159, 2164-2171. Assays can be performed either using a modification of the "batch" method of W.J. Thompson et al. (Biochem. (1979) 18, 5228) or using a scintillation proximity assay for the direct detection of AMP/GMP using a modification of the protocol described by Amersham pic under product code TRKQ7090/7100. Their functional activity can be assessed *In vitro* by determining the capacity of a PDE5 inhibitor of the invention to enhance sodium nitroprusside or electric field stimulation-induced relaxation of pre-contracted rabbit corpus cavemosum tissue strips, as described by S.A. Ballard et al. ((1998) J. Urology 159, 2164-2171).

Another aspect of the invention is the use of a combination of a vasopressin V1a receptor antagonist with a PDE V inhibitor for the preparation of a medicament for the treatment of premature ejaculation or rapid ejaculation.

Yet another aspect of the Invention is the use of a combination of a vasopressin V1a receptor antagonist with a selective serotonin reuptake inhibitor (SSRI) for the preparation of a medicament for the treatment of premature ejaculation or rapid ejaculation.

A further aspect of the invention are products containing a vasopressin V1a receptor antagonist and a PDE V inhibitor as a combined preparation for simultaneous, separate or sequential use in treating premature ejaculation or rapid ejaculation.

Yet a further aspect of the invention are products containing a vasopressin V1a receptor antagonist and a selective serotonin reuptake inhibitor (SSRI) for the preparation of a medicament for the treatment of premature ejaculation or rapid ejaculation.

Reference to an antagonist, an agonist or an inhibitor shall at all times be understood to include all active forms of such agents, including the free form thereof (e.g. the free and/or base form) and also all pharmaceutically acceptable salts, polyrnorphs, hydrates, silicates, stereo-isomers (e.g. diastereolsomers and enantiomers) and so forth. Active metabolites of any of the compounds, in any form, are also included.

Particular formulations of the compounds for either oral delivery or for topical application (creams, gels) are Included in the Invention.

The antidiuretic hormone vasopressin is a cyclic nonapeptide involved in the control of body fluid osmolality, blood volume, blood pressure, and vascular tone. It acts by binding to G protein-coupled membrane receptors. One member of this receptor family is the vasopressin V1a receptor, which mediates cell contraction and proliferation, platelet aggregation, release of coagulation factor, and glycogenolysis. Vasopressin (AVP) action through the vasopressin V1a receptor is mediated by activating phospholipase C, which in turn stimulates phosphatidylinositol turnover to Increase intracellular calcium ion. Vasopressin V1a receptors were first cloned from rat from liver cDNA (Morel, A. et al (1992) Nature 356, 523-526), and the sequence was deposited In Genbank with accession number Z11690. The human vasopressin V1a receptor was cloned and functionally expressed by Thibonnier et al ((1994) J. Bioi.Chem. 269, 3304-3310), and the sequence was deposited in Genbank with accession number L25615. The protein sequence of the human vasopressin V1a receptor is also available in SwissProt accession P37288.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "protein".

In addition to the specific amino acid sequences mentioned herein, the present Invention also encompasses the use of variants, homologues and derivatives thereof.

In the present context, a homologous sequence is taken to include an amino acid sequence which may be at least 75, 80, 85 or 90% identical to the amino acid sequence of the human V1a receptor shown in Thibonnier, M. et al ((1994) J. Biol. Chem. 269, 3304-3310), preferably at least 95 or 98% identical. In particular, homology should typically be considered with respect to those regions of the sequence known to be essential for an activity. Although homology can also be considered in terms of similarity (i.e. amino acid residues having similar chemical properties/functions), in the context of the present Invention it is preferred to express homology in terms of sequence identity. Such sequence homology/identity can be easily assessed by publicly or commercially available bioinformatics software, such as Blast2 (Altschul, S.F. et al (1997) Nucl. Acids Res. 25, 3389-3402), or programs included in the GCG software package (Devereux et al (1984) Nucl. Acids Res. 12, 387; Wisconsin Package Version 10, Genetics Computer Group (GCG, Madison, Wisconsin), such as Bestfit or Gap. In most cases, the default parameters offered by the software, e.g. Bestfit or Gap, for Gap Penalties etc. are suitable for this assessment.

"Potency" as used herein is a measure of the concentration of a compound at which it is effective. The potency of a compound can be determined in a binding assay as described In Example 2, and potency in this context will refer to the IC₅₀ of the compound, i.e. to the concentration inhibiting 50% of the labelled compound from binding to the receptors. The potency of a compound can also be determined In a functional assay such as delay of ejaculation in anaesthetlsed rats as described in Example 1. The potency in this case would refer to the IC₅₀ of the compound, i.e. the concentration which inhibits 50% of the functional response seen by application of the agonist.

"Selectivity" as used herein is a measure of the relative potency of a drug between two receptor subtypes for the same endogenous ligand. This can be determined in binding assays as described in Example 2, or in functional assays as described in Example 3.

For the avoidance of doubt, the term "compound" may refer to a chemical or biological agent, and Includes, for example, antibodies, antibody fragments, other proteins, peptides, sugars, any organic or inorganic molecules. Compounds that may be used for screening Include, but are not limited to, peptides such as, for example, soluble peptides, including but not limited to members of random peptide libraries; (see, e.g., Lam et al. (1991) Nature 354, 82-84; Houghten et al. (1991) Nature 354, 84-86), and combinatorial chemistry-derived molecular library made of D- and/or L- configuration amino acids, phosphopeptides (including, but not limited to, members of random or partially degenerate, directed phosphopeptide libraries; see, e.g., Songyang et al. (1993) Cell 72, 767-778), antibodies (including, but not limited to, polyclonal, monoclonal, humanized, anti-idlotypic, chimeric or single chain antibodies, and Fab, F(ab')₂ and Fab expression library fragments, and apitope-binding fragments thereof), and small organic or inorganic molecules.

The suitability of the vasopressin V1a receptor antagonist can be readily determined by evaluation of their potency and selectivity using methods such as those disclosed herein, followed by evaluation of their toxicity, pharmacokinetics (absorption, metabolism, distribution and elimination), etc in accordance with standard pharmaceutical practice. Suitable compounds are those that are potent and selective, have no significant toxic effect at the therapeutic dose, and preferably are bioavailable following oral administration.

Oral bioavailablity refers to the proportion of an orally administered drug that reaches the systemic circulation. The factors that determine oral bioavailability of a drug are dissolution, membrane permeability and hepatic clearance. Typically, a screening cascade of firstly *in vitro* and then *in vivo* techniques is used to determine oral bioavailablity.

Dissolution, the solubilisation of the drug by the aqueous contents of the gastro-intestinal tract (GIT), can be predicted from in vitro solubility experiments conducted at appropriate pH to mimic the GIT. Preferably the vasopressin V1a receptor antagonists have a minimum solubility of 50µg/ml. Solubility can be determined by standard procedures known in the art such as described In Lipinski CA et al.; Adv. Drug Deliv. Rev. 23(1-3), 3-25,1997.

Membrane permeability refers to the passage of a compound through the cells of the GIT. Lipophilicity is a key property in predicting this and is determined by *in vitro* Log D_{7.4} measurements using organic solvents and buffer. Preferably the vasopressin V1a receptor antagonists have a Log D_{7.4} of -2 to +4, more preferably -1 to +3. The Log D can be determined by standard procedures known in the art such as described in Stopher, D and McClean, S; J. Pharm. Pharmacol. 42(2), 144, 1990.

Cell monolayer assays such as Caco2 add substantially to prediction of favourable membrane permeability in the presence of efflux transporters such as P-glycoprotein, so-called Caco2 flux. Preferably, the vasopressin V1a receptor antagonists have a Caco2 flux of greater than 2x10⁻⁶cms⁻¹, more preferably greater than 5x10⁻⁶cms⁻¹. The Caco2 flux value can be determined by standard procedures known in the art such as described in Artursson, P and Magnusson, C; J. Pharm. Sci, 79(7), 595-600, 1990.

Metabolic stability addresses the ability of the GIT to metabolise compounds during the absorption process or the liver to do so immediately post-absorption: the first pass effect. Assay systems such as microsomes, hepatocytes etc are predictive of metabolic lability. Preferably vasopressin V1a receptor antagonists show metabolic stability in the assay system that is commensurate with an hepatic extraction of less then 0.5. Examples of assay systems and data manipulation are described in Obach, RS; Curr. Opin. Drug Disc. Devel. 4(1), 36-44, 2001 and Shibata, Y et al.; Drug Met. Disp. 28(12), 1518-1523, 2000.

Because of the interplay of the above processes, further support that a drug will be orally bioavailable in humans can be gained by *in vivo* experiments in animals. Absolute bioavailabillty is determined in these studies by administering the compound separately or in mixtures by the oral route. For absolute determinations (% orally bioavailable) the intravenous route is also employed. Examples of the assessment of oral bioavailability In animals can be found In Ward, KW et al.; Drug Met. Disp. 29(1), 82-87, 2001; Berman, J et al.; J. Med. Chem. 40(6), 827-829, 1997 and Han KS and,Lee, MG; Drug Met. Disp. 27(2), 221-226, 1999.

The compounds of the invention can be administered alone but will generally be administered in admixture with a suitable pharmaceutical excipient, diluent or carrier selected with regard to the intended route of administration and standard pharmaceutical practice.

For example, the compounds of the Invention can be administered orally, buccally or sublingually in the form of tablets, capsules, multi-particulates, gels, films, ovules, elixirs, solutions or suspensions, which may contain flavouring or colouring agents, for immediate-, delayed-, modified-, sustained-, pulsed- or controlled-release applications. The compounds of the invention may also be administered as fast-dispersing or fast-dissolving dosage forms or in the form of a high energy dispersion or as coated particles. Suitable formulations may be in coated or uncoated form, as desired.

Such solid pharmaceutical compositions, for example, tablets, may contain excipients such as microcrystalline cellulose, lactose, sodium citrate, calcium carbonate, dibasic calcium phosphate, glycine and starch (preferably com, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

The following formulation examples are illustrative only and are not intended to limit the scope of the invention. Active ingredient means a compound of the invention.

### Formulation 1:

A tablet is prepared using the following ingredients :
Active ingredient (50mg) is blended with cellulose (microcrystalline), silicon dioxide, stearic acid (fumed) and the mixture is compressed to form tablets.

### Formulation 2:

An intravenous formulation may be prepared by combining active ingredient (100mg) with isotonic saline (1000ml)

The tablets are manufactured by a standard process, for example, direct compression or a wet or dry granulation process. The tablet cores may be coated with appropriate overcoats.

Solid compositions of a similar type may also be employed as fillers In gelatin or HPMC capsules. Preferred excipients in this regard include lactose, starch, a cellulose, milk sugar or high molecular weight polyethylene glycols. For aqueous suspensions and/or elixirs, the V1a receptor antagonists may be combined with various sweetening or flavouring agents, colouring matter or dyes, with emulsifying and/or suspending agents and with diluents such as water, ethanol, propylene glycol and glycerin, and combinations thereof.

Modified release and pulsatile release dosage forms may contain excipients such as those detailed for Immediate release dosage forms together with additional excipients that act as release rate modifiers, these being coated on and/or included in the body of the device. Release rate modifiers include, but are not exclusively limited to, hydroxypropylmethyl cellulose, methyl cellulose, sodium carboxymethylcellulose, ethyl cellulose, cellulose acetate, polyethylene oxide, Xanthan gum, Carbomer, ammonio methacrylate copolymer, hydrogenated castor oil, carnauba wax, paraffin wax, cellulose acetate phthalate, hydroxypropylmethyl cellulose phthalate, methacrylic acid copolymer and mixtures thereof. Modified release and pulsatile release dosage forms may contain one or a combination of release rate modifying excipients. Release rate modifying excipients may be present both within the dosage form i.e. within the matrix, and/or on the dosage form, i.e. upon the surface or coating.

Fast dispersing or dissolving dosage formulations (FDDFs) may contain the following Ingredients: aspartame, acesulfame potassium, citric acid, croscarmellose sodium, crospovidone, diascorbic acid, ethyl acrylate, ethyl cellulose, gelatin, hydroxypropylmethyl cellulose, magnesium stearate, mannitol, methyl methacrylate, mint flavouring, polyethylene glycol, fumed silica, silicon dioxide, sodium starch glycolate, sodium stearyl fumarate, sorbitol, xylitol. The terms dispersing or dissolving as used herein to describe FDDFs are dependent upon the solubility of the drug substance used i.e. where the drug substance is Insoluble a fast dispersing dosage form can be prepared and where the drug substance is soluble a fast dissolving dosage form can be prepared.

The compounds of the Invention can also be administered parenterally, for example, intracavernouslly, Intravenously, intra-arterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The following dosage levels and other dosage levels herein are for the average human subject having a weight range of about 65 to 70kg. The skilled person will readily be able to determine the dosage levels required for a subject whose weight falls outside this range, such as children and the elderly.

The dosage of the combination of the Invention in such formulations will depend on its potency, but can be expected to be In the range of from 1 to 500mg of vasopressin V1a receptor antagonist for administration up to three times a day. A preferred dose is in the range 10 to 100mg (e.g. 10, 25, 50 and 100mg) of vasopressin V1a receptor antagonist which can be administered once, twice or three times a day (preferably once). However the precise dose will be as determined by the prescribing physician and will depend on the age and weight of the subject and severity of the symptoms.

For oral and parenteral administration to human patients, the daily dosage level of a compound of the invention will usually be from to 5 to 500mg/kg (in single or divided doses).

Thus tablets or capsules may contain from 5mg to 250mg (for example 10 to 100mg) of the compound of the invention for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention. The skilled person will appreciate that the compounds of the invention may be taken as a single dose as needed or desired (i.e. prn). It is to be appreciated that all references herein to treatment include acute treatment (taken as required) and chronic treatment (longer term continuous treatment).

The compounds of the invention can also be administered intranasally or by inhalation and are conveniently delivered in the form of a dry powder inhaler or an aerosol spray presentation from a pressurised container, pump, spray, atomiser or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of the compounds of the invention and a suitable powder base such as lactose or starch.

Aerosol or dry powder formulations are preferably arranged so that each metered dose or "puff" contains from 1µg to 50mg of a compound of the invention for delivery to the patient. The overall daily dose with an aerosol will be in the range of from 1µg to 50mg which may be administered in a single dose or, more usually, in divided doses throughout the day.

Alternatively, the compounds of the invention can be administered in the form of a suppository or pessary, or they may be applied topically in the form of a gel, hydrogel, lotion, solution, cream, ointment or dusting powder. The compounds of the Invention may also be dermally or transdermally administered, for example, by the use of a skin patch, depot or subcutaneous injection. They may also be administered by the pulmonary or rectal routes.

For application topically to the skin, the compounds of the Invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved In, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds of the Invention may also be used In combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in published international patent applications WO91/11172, WO94/02518 and WO98/55148.

Oral administration of the compounds of the invention is a preferred route, being the most convenient. In circumstances where the recipient suffers from a swallowing disorder or from impairment of drug absorption after oral administration, the drug may be administered parenterally, sublingually or buccally.

### Examples

The examples below are carried out using standard techniques, which are well-known and routinely used by those skilled in the art; the examples illustrate but do not limit the invention.

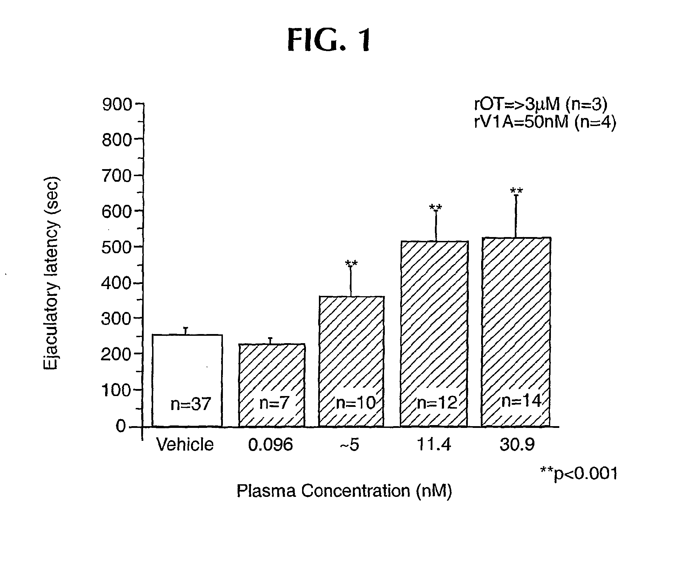

Figure 1: Compound 1 (4-[4-Benryl-5-(4-methoxy-piperidin-1-ylmethyl)-4H-[1,2,4]triazoi-3-yl]-3,4,5,6-tetrahydro-2H-[1,2']bipyridinyl) delays ejaculation in an anaesthetised rodent model of ejaculation.

### Example 1: Vasopressin V1a antagonists delay ejaculation In anaesthetised rats

In order to study penile erection and ejaculation the method used was based on the methodology taught in Yonezawa et al (2000) Life Sciences 67, 3031-3039. For ease of reference, this methodology is described below:

Male Sprague Dawley rats, weighing 350-450 g, are used. Prior to the experiments the animals are housed in groups (2 rats per cage) under controlled 12 h light-dark cycle (lights on at 07:00), constant temperature (23±1°C) and humidity (55±5%). They have free access to standard food pellets and water.

Rats are anesthestised with sodium pentobarbitone (50mg/kg, i.p.) and are placed in the supine position. The penis is extruded from its sheath and gently held by a wooden applicator positioned at the base of the penis. The test compounds are administered by intravenous infusion and p-chloroamphetamine (PCA) (5-10mg/kg) is administered i.p. immediately before the sheath retraction and the penile responses, including penile erection, redding and expansion of the penile body, glans erection, engorgement and slight flaring of the glans and cup, glans erection with Intense flaring of the glans, are recorded. Latencies from PCA administration to the initial penile response and ejaculation is also measured In seconds.

The effect of a test compound on p-chloroamphetamine (PCA) induced ejaculation is also assessed by weighing the ejaculates accumulated over 30mins. A suitable method using conscious rats is described in Renyl (1985) Neuropharmacology, Vol. 24. No. 8, pp 697-704.

Intracavemosal pressure is also determined in rats anesthetised with sodium pentobarbitone (50 mg/kg, i.p.). Further small additional amounts (5mg) may be injected throughout the experimental period as required. The penis is extruded from its sheath and the intracavernosal pressuer (ICP) was measured by inserting a stainless steel needle (23-guauge) into one corpus cavernosum. The needle is attached to a heparinized saline (10U/ml)-fllled teflon tube and connected to a pressure transducer (NEC-San-Ei 7500).

For all the sexual behavour tests, the male rats were placed in an observation arena (50-60 cm diameter), starting 5 hours into the dark cycle and observed under red ilumination. Three to four minutes after placing the male in the arena, a receptive female (ovariectomised, oestradiol benzoate / progesterone injection 48 hour before behavioural study) was Introduced into the arena and the following parameters noted:
i) ejaculatory latency (EJL; time taken from addition of receptive female into the arena to ejaculation);
ii) copulatory efficiency (CE; ejaculatory latency/ the number of intromissions to ejaculation, i.e. the number of seconds between intromissions);
iii) intromission frequency (IF; the number of intromissions to ejaculation);
iv) mount frequency (MF; the number of mounts to ejaculation);
v) post ejaculatory interval (PEI; the time taken from ejaculation to the commencement of copulatory behaviour).

The compound used in the following Examples was as follows:

Compound 1 (Example 26 in WO 04/037809: 4-[4-Benzyl-5-(4-methoxy-piperldin-1-ylmethyl)-4H-[1,2,4]triazol-3-yl]-3,4,5,6-tetrahydro-2H-[1,2']bipyridinyl); this compound is more than 60-fold selective towards vasopressin V1a receptors as compared with oxytocin receptors [50nM V1a vs 3 µM OT].

Selective vasopressin receptor antagonist L-371,257. Further details regarding this compound are provided In Williams PD et al (1995) J Med Chem; 38: 4634-4636. L-371,257 is more than 5-fold selective towards oxytocin receptors as compared with vasopressin V1a receptors [3.7nM V1a vs 19 nM OT :].

### Example 1a. Delaying ejaculation in the presence of a selective vasopressin V1A receptor antagonist (Compound 1)

A vasopressin V1A receptor antagonist, compound 1, significantly delayed p-chloroamphetamine (PCA)-induced ejaculation at vasopressin selective doses In anaesthetised rats (plasma concentration of 9-31 nM). Ejaculation was delayed 100% (near maximal effect) at free plasma concentrations 30.9nM (0.6xKi V1a, see Figure 1) - it has been assumed that at these doses any activity arises from antagonism of vasopressin V1a receptors since at the plasma concentration at which the compound delayed ejaculation, compound 1 would display minimal if any activity at oxytocin receptors (<0.01Kᵢ OT).

Erectogenic mechanisms were largely unaffected by vasopressin V1 a receptor blockade - the time taken for the rats to achieve erection was not significantly affected by compound 1 neither was the quality of penile erection - the number of penile cups and flares was similar in control and vasopressin V1A antagonist studies (see Table 1 below). At plasma concentrations of 30.9nM compound 1 (a dose that significantly delays ejaculation) - 84% of PCA-induced erections resulted in penile flares compare to 82% in vehicle control groups and 57% of PCA-induced erections resulted in penile cups compare to 32% in vehicle control groups.

**Table 1:**

| | | Compound 1 plasma conc | |
|---|---|---|---|
| | Vehicle | 11.4nM * | 30.9nM * |
| Penile Cups | 36.9% | 31.6% | 57.0% |
| Penile Flares | 85.1% | 81.5% | 83.6% |

| | | | |
|---|---|---|---|
| * At both of these concentrations Compound 1 delayed ejaculation without affecting penile erection. | | | |

Using a rodent model of ejaculation, that reflects human ejaculatory physiology, we have shown that that vasopressin V1a receptors are involved in the ejaculatory mechanisms.

Moreover, the study shows that a vasopressin V1a receptor antagonist will be useful in the treatment of premature ejaculation by delaying ejaculation.

### Example 1b: Effect of a vasopressin V1a receptor antagonist (L-371257) on copulatory behaviour in rats

Rodent copulatory behaviour is characterised by a series of mounts, with and without vaginal insertion (50-80% of mounts result in intromission [vaginal penetration]) and ejaculation occurs after 6 to 12 intromissions. Each intromission lasts a matter of seconds - it is not possible to quantify intromission length i.e. intravaginal latency. The effect of L-371257 was assessed on a number of copulatory parameters (see above). We have focused ejaculatory latency as a clinical biomarker of time taken to achieve ejaculation.

L-371257, a V1a antagonist, Increased ejaculatory latency by 67% In conscious rats (P<0.05); i.e. L-371257-treated animals took 266s to ejaculate compared to 160s In vehicle treated animals (see Table 2 below). There were no other significant effects of on copulatory behaviour. At the dose tested, L-371257 is likely to be selective for vasopressin V1a receptors (See example 1a).

**Table 2:**

| | Vehicle | L-371257 0.05mgkg⁻¹ sc Plasma conc ∼ 30nM |
|---|---|---|
| Ejaculatory latency (sec) | 159.6 ± 16.7s | 266.4 ± 30.9s P<0.05 |

| | | |
|---|---|---|
| mean ± sem (n=14, 8, respectively). | | |

Using a conscious rodent model of copulatory behaviour to assess ejaculatory latency, that reflects human ejaculatory physiology, we have shown that that vasopressin V1a receptors are involved In the ejaculatory mechanisms. Moreover, the study shows that a vasopressin V1a antagonist will be useful In the treatment of premature ejaculation by delaying ejaculation.

### Example 2: Ligand binding assay

Receptor binding assays were performed on cellular membranes prepared from CHO cells stably expressing the human V_{1A} receptor, (CHO-hV_{1A}). The CHO-hV_{1A} cell line was kindly provided under a licensing agreement by Marc Thibonnier, Dept. of Medicine, Case Western Reserve University School of Medicine, Cleveland, Ohio. CHO-hV_{1A} cells were routinely maintained at 37°C In humidified atmosphere with 5% CO₂ in DMEM/Hams F12 nutrient mix supplemented with 10 % fetal bovine serum, 2 mM L-glutamine, 15 mM HEPES and 400 µg/ml G418. For bulk production of cell pellets, adherent CHO-hV_{1A} cells were grown to confluency of 90-100% in 850 cm² roller bottles containing a medium of DMEM/Hams F12 Nutrient Mix supplemented with 10 % fetal bovine serum, 2 mM L-glutamine and 15 mM HEPES. Confluent CHO-hV_{1A} cells were washed with phosphate-buffered saline (PBS), harvested into ice cold PBS and centrifuged at 1,000 rpm. Cell pellets were stored at -80°C until use. Cell pellets were thawed on ice and homogenised in membrane preparation buffer consisting of 50 mM Tris-HCI, pH 7.4, 5 mM MgCl₂ and supplemented with a protease inhibitor cocktail, (Roche). The cell homogenate was centrifuged at 1000 rpm, 10 min, 4°C and the supernatant was removed and stored on ice. The remaining pellet was homogenised and centrifuged as before. The supernatants were pooled and centrifuged at 25,000 x g for 30 min at 4°C. The pellet was resuspended in freezing buffer consisting of 50 mM Tris-HCI, pH 7.4, 5 mM MgCl₂ and 20 % glycerol and stored in small aliquots at -80°C until use. Protein concentration was determined using Bradford reagent and BSA as a standard.

Protein linearity followed by saturation binding studies were performed on each new batch of membrane. A membrane concentration was chosen that gave specific binding on the linear portion of the curve. Saturation binding studies were then performed using various concentrations of [³H]-arginine vasopressin, [³H]-AVP (0.05 nM - 100 nM) and the *K*_{d} and Bₘₐₓ determined.

Compounds were tested for their effects on [³H]-AVP binding to CHO-hV_{1A} membranes, (³H-AVP; specific activity 65.5 Ci/mmol; NEN Life Sciences). Compounds were solubilised In dimethylsulfoxide (DMSO) and diluted to working concentration of 10% DMSO with assay buffer containing 50 mM Tris-HCL pH 7.4, 5 mM MgCl₂ and 0.05% BSA. 25 µl compound and 25 µl [³H]-AVP, (final concentration at or below *K*_{d} determined for membrane batch, typically 0.5 nM - 0.6 nM) were added to a 96-well round bottom polypropylene plate. The binding reaction was initiated by the addition of 200 µl membrane and the plates were gently shaken for 60 min at room temperature. The reaction was terminated by rapid filtration using a Filtermate Cell Harvester (Packard Instruments) through a 96-well GF/B UniFilter Plate which had been presoaked in 0.5% polyethyleneimine to prevent peptide sticking. The filters were washed three times with 1 ml ice cold wash buffer containing 50 mM Tris-HCL pH 7.4 and 5 mM MgCl₂. The plates were dried and 50 µl Microscint-0 (Packard Instruments) was added to each well. The plates were sealed and counted on a TopCount Microplate Scintillation Counter (Packard Instruments). Non-specific binding (NSB) was determined using 1 µM unlabelled d(CH2)5Tyr(Me)AVP ([β-mercapto-β,β-cyclopentamethylene-propionyl,0-Me-Tyr²,Arg⁸]-vasopressin) (βMCPVP), (Sigma). The radioligand binding data was analysed using a four parameter logistic equation with the min forced to 0%. The slope was free fitted and fell between -0.75 and -1.25 for valid curves. Specific binding was calculated by subtracting the mean NSB cpm from the mean Total cpm. For test compounds the amount of ligand bound to the receptor was expressed as % bound = (sample cpm - mean NSB cpm)/specific binding cpm x100. The % bound was plotted against the concentration of test compound and a sigmoidal curve was fitted. The Inhibitory dissociation constant (*K*ᵢ) was calculated using the Cheng-Prusoff equation: *K*ᵢ=IC₅₀/(1+[L]/*K*_{d}) where [L] is the concentration of ligand present in the well and *K*_{d} is the dissociation constant of the radioligand obtained from Scatchard plot analysis.

### Example 3: Functional assay: inhibition of AVP / V_{1A}-R mediated Ca²⁺ mobilization by FLIPR (Fluorescent Imagine Plate Reader) (Molecular Devices)

Intracellular calcium release was measured in CHO-hV_{1A} cells using FLIPR, which allows the rapid detection of calcium following receptor activation. The CHO-hV_{1A} cell line was maintained as described in Example 2 above. On the afternoon before the assay cells were plated at a density of 20,000 cells per well into black sterile 96-well plates with clear bottoms to allow cell inspection and fluorescence measurements from the bottom of each well. Wash buffer containing Dulbecco's phosphate buffered saline (DPBS) and 2.5 mM probenecid and loading dye consisting of cell culture medium containing 4 µM Fluo-3-AM (dissolved in DMSO and pluronic acid, Molecular Probes) and 2.5 mM probenecid was prepared fresh on the day of assay. Compounds were solubilised in DMSO and diluted In assay buffer consisting of DPBS containing 1 % DMSO, 0.1 % BSA and 2.5 mM probenecid. The cells were incubated with 100 µl loading dye per well for 1 hour at 37°C in humidified atmosphere with 5% CO₂. After dye loading the cells were washed three times in 100 µl wash buffer using a Denley plate washer. 100 µl wash buffer was left in each well. Intracellular fluorescence was measured using FLIPR. Fluorescence readings were obtained at 2s intervals with 50 µl of the test compound added after 30s. An additional 155 measurements at 2s Intervals were then taken to detect any compound agonistic activity. 50 µl of arginine vasopressin (AVP) was then added so that the final assay volume was 200 µl. Further fluorescence readings were collected at 1s intervals for 120s. Responses were measured as peak fluorescence intensity (FI). For pharmacological characterization a basal Fl was subtracted from each fluorescence response. For AVP dose response curves, each response was expressed as a % of the response to the highest concentration of AVP in that row. For IC₅₀ determinations , each response was expressed as a % of the response to AVP. IC₅₀ values were converted to a modified *K*_{b} value using the Cheng-Prusoff equation which takes into account the agonist concentration, [A], the agonist EC₅₀ and the slope: *K*_{b}=IC₅₀/(2+[A]/A₅₀]ⁿ)^{1/n}-1 where [A] is the concentration of AVP, A₅₀ is the EC₅₀ of AVP from the dose response curve and n=slope of the AVP dose response curve.

### Example 4: Oxytocin receptor binding assay

As an example for a selectivity assay, an oxytocin receptor binding assay is described below.

CHO-cells engineered to express the human Oxytocin receptor are maintained, and membrane preparations from such cells are prepared according to standard techniques (see e.g. Example 2). They are diluted to 1 mg/ml protein concentration in assay buffer (50 mM Tris-HCl, pH7.8; 10 mM Mg Cl₂; 0.25% BSA). SPA beads are resuspended at 50 mg/ml in assay buffer. From these concentrations, beads are pre-coupled with membranes by incubating 30 µg of protein per mg of bead on a top-to-tail shaker for 2 hours at 4 °C. The bead/membranes are then centrifuged at 2000 rpm for 10 mins and the pellet is resuspended at 3 mg/ml.

All manipulations of the ¹²⁵I-OVT (NEN, NEX254) are carried out using tips that have been silanised using SigmaCote. All bottles and tubes are also silanised. The ¹²⁵I-OVT is diluted in 1ml assay buffer per 50 µCi of lyophilised ligand. A 5 µl sample is then counted in duplicate using liquid scintillation counting (protocol 61 on Wallac Counter) and the concentration of the ligand is calculated. This is to overcome any loss of ligand due to stickiness. Using the measured concentration, the ¹²⁵I-OVT is diluted to 0.3 nM in assay buffer.

20 µl of the bead/membrane preparation is added to the prepared Optiplates using the Multi-drop, after the desired dilutions of test compounds are added to the wells. The bead/membrane preparation is kept in suspension using a stirring flask. 20 µl of the ¹²⁵I-OVT is then added to each well of the Optiplate using the Multi-drop. Following a 4 hour incubation at room temperature, the plates are counted using the TopCount NXT for 30s/well.

The skilled person will be able to adapt the above ligand binding assay, as well as the functional assay of Example 3, for other receptors such as Oxytocin receptor, V2 vasopressin receptor and vasopressin V1b receptor.

## Claims

1. Use of a compound of formula (Ia): 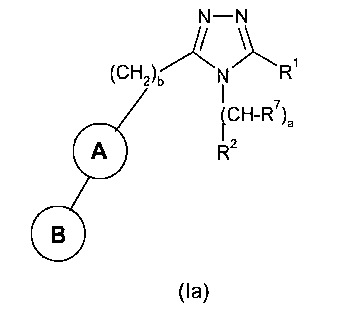 or a pharmaceutically acceptable salt or solvate thereof, wherein
R¹ represents C₁-C₆ alkyl, -(CH₂)_{c}-[C₃-C₈ cycloalkyl]-, -(CH₂)_{c}-W or-(CH₂)_{c}-Z-(CH₂)_{d}-W;
R² represents a phenyl group, optionally fused to a 5- or 6- membered aryl or heterocyclic group which may contain one or more heteroatoms selected from N, O or S; the phenyl group and the optionally fused group being optionally substituted with one or more groups independently selected from the list defined below;
Ring **A** represents a 4-, 5- or 6- membered saturated heterocyclic group containing at least one N;
Ring **B** represents a phenyl group or het¹, each group being optionally substituted with one or more groups independently selected from the list defined below;
het ¹ represents a 4-, 5- or 6- membered saturated, or unsaturated, heterocyclic group containing at least one N (but which may also contain one or more O or S atoms);
R⁷ independently represents H, C₁-C₆ alkyl, OR³, -(CH₂)ₑ-R³ or -(CH₂)_{f}-O-(CH₂)ₑ-R³;
W represents a phenyl group, NR⁴R⁵ or het², the phenyl group being optionally substituted with one or more groups independently selected from halogen, CF₃, OCF₃, R³, OR³, CO₂R³, CONR⁴R⁵, CN, SO₂NR⁴R⁵ and NR³SO₂Me;
het² represents a 4-, 5-, 6- or 7- membered saturated, or unsaturated, heterocyclic group containing at least one N (but which may also contain one or more O or S atoms), optionally substituted with one or more groups independently selected from the list defined below;
Z represents O or S(O)g;
g represents 0, 1 or 2;
het³ represents a 4-, 5-, 6- or 7- membered saturated or unsaturated heterocyclic group containing at least one N (but which may also contain one or more O or S atoms), optionally substituted with one or more groups independently selected from the list defined below;
at each occurrence R³ and R⁶ independently represent H, C₁-C₆ alkyl optionally substituted by Y, -(CH₂)_{g}-[C₃-C₈ cycloalkyl], phenyl, benzyl, pyridyl or pyrimidyl;
Y independently represents a phenyl group, NR⁴R⁵ or het³, the phenyl group being optionally substituted with one or more groups independently selected from halogen, CF₃, OCF₃, R⁴, OR⁴, CO₂R⁴, CONR⁴R⁵, CN, SO₂NR⁴R⁵, NR⁴SO₂Me and -NR⁴R⁵;
at each occurrence R⁴ and R⁵ independently represent H, C₁-C₆ alkyl, -(CH₂)g-[C₃-C₈ cycloalkyl], phenyl, benzyl, pyridyl or pyrimidyl; or R⁴ and R⁵ together with the N atom to which they are attached represent a heterocyclic group of from 3 to 8 atoms;
substituents for R², Ring B, het¹, het² and het³ are independently selected from the following list: halogen, CF₃, OCF₃, R³, -(CH₂)ₑ-OR³, -(CH₂)ₑ-CO₂R³, -(CH₂)ₑ-CONR⁴R⁵, - (CH₂)ₑ-CN, - (CH₂)ₑ-SO₂NR⁴R⁵, -(CH₂)ₑ-NR³SO₂Me, -(CH₂)ₑ-COR³, -(CH₂)ₑ-OCOR³, -(CH₂)ₑ-NHCOR³, - (CH₂)ₑ-NR³COR⁶ and -(CH₂)ₑNR⁴R⁵;
a and b independently represent 0 or 1;
c, d, e and g independently represent 0, 1, 2, 3 or 4;
f independently represents 1, 2, 3 or 4;
provided that a + b cannot equal 0; and
provided that when R¹ represents -(CH₂)_{c}-Z-(CH₂)_{d}-W and W represents NR⁴R⁵ or any N linked heterocyclic group then d must not be 0 or 1; and
provided that when R² represents a phenyl group substituted by a group of formula - (CH₂)ₑOR³, -(CH₂)ₑ-CO₂R³ or -(CH₂)ₑOCOR³; or
het¹ and/or het² are substituted by a group of formula -(CH₂)ₑOR³, -(CH₂)ₑ-CO₂R³ or - (CH₂)ₑOCOR³; or
when R⁷ represents -OR³ or -(CH₂)_{f}O-(CH₂)ₑ-R³ and e is 0; or
when W represents a phenyl group substituted with -OR³ or -CO₂R³;
and R³ represents an alkyl group substituted with Y, and Y represents NR⁴R⁵ or an N-linked het³;
then R³ must represent C₂-C₆ alkyl substituted with Y,
in the manufacture of a medicament for the treatment of premature ejaculation.

2. The use according to claim 1, wherein the compound of formula (Ia) is 4-[4-Benzyl-5-(4-methoxy-piperidin-1-ylmethyl)-4*H*-[1,2,4]triazol-3-yl]-3,4,5,6-tetrahydro-2*H*-[1,2']bipyridinyl or a pharmaceutically acceptable salt thereof.

3. Use of a compound of formula (I): 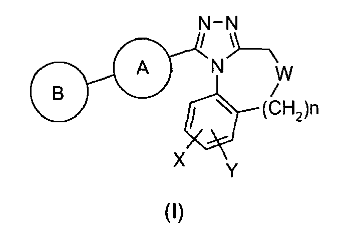 or a pharmaceutically acceptable salt or solvate thereof, wherein
W is O, S, or NR¹
R¹ represents H, C₁₋₆ alkyl, -(CH₂)ₐ-[C₃₋₈ cycloalkyl], phenyl, benzyl, pyridyl, pyrimidyl, -COR², -CO₂R², -CO-(CH₂)ₐ-NR²R³, -SO₂R², -(CH₂)_{b}-OR², -(CH₂)_{b}-NR²R³, or a saturated heterocycle of from 3 to 8 atoms containing one or more heteroatoms selected from O, N and S;
X and Y independently represent H, halogen, OH, CF₃, OCF₃, R⁴, -(CH₂)_{d}-CONR⁴R⁵, - (CH₂)_{d}-CN, -(CH₂)_{d}-SO₂NR⁴R⁵, -(CH₂)_{d}-NR⁴SO₂Me, -(CH₂)_{d}-COR⁴, -(CH₂)_{d}-OCOR⁴, -(CH₂)_{d}-NHCOR⁴, -(CH₂)_{d}-NR⁴COR⁵, -(CH₂)_{d}-OR⁶or -(CH₂)_{d}-CO₂R⁶;
Ring **A** represents a piperidinyl, piperazinyl, pyrrolidinyl or azetidinyl group;
Ring **B** represents a phenyl, pyridinyl or pyrimidinyl group (optionally substituted with one or more groups independently selected from halogen, CN, CONH₂, CF₃, OCF₃, R⁷, and -(CH₂)_{f}-OR⁸);
R², R³, R⁴, R⁵ and R⁷ independently represent H, straight or branched C₁₋₆ alkyl, -(CH₂)_{c}-[C₃₋₈ cycloalkyl], phenyl, benzyl, pyridyl or pyrimidyl;
or R² and R³, or R⁴ and R⁵, together with the nitrogen atom to which they are attached independently represent a heterocycle of from 3 to 8 atoms;
R⁶ and R⁸ independently represent H, straight or branched C₁₋₆ alkyl, -(CH₂)_{c}-[C₃₋₈ cycloalkyl], -(CH₂)ₑ-NR⁴R⁵,-(CH₂)ₑ-OR⁴, phenyl, benzyl, pyridyl or pyrimidyl;
n = 0, 1 or 2;
a , c, d and f are each independently selected from 0, 1, 2 and 3;
b and e are each independently selected from 2 and 3,
in the manufacture of a medicament for the treatment of premature ejaculation or rapid ejaculation.

4. The use according to claim 3, wherein the compound of formula (I) is selected from:
1-(3,4,5,6-Tetrahydro-2H-[1,2']bipyridinyl-4-yl)-5,6-dihydro-4H-2,3,5,10b-tetraaza-benzo[e]azulene;
5-Methyl-1-(3,4,5,6-tetrahydro-2H-[1,2']bipyridinyl-4-yl)-5,6-dihydro-4H-2,3,5,10b-tetraaza-benzo[e]azulene;
1-[1-(3,4,5,6-Tetrahydro-2H-[1,2']bipyridinyl-4-yl)-4H,6H-2,3,5,10b-tetraaza-benzo[e]azulen-5-yl]-ethanone;
8-Chloro-1-(3,4,5,6-tetrahydro-2H-[1,2']bipyridinyl-4-yl)-5,6-dihydro-4H-2,3,5,10b-tetraaza-benzo[e]azulene;
8-Chloro-5-methyl-1-(3,4,5,6-tetrahydro-2H-[1,2']bipyridinyl-4-yl)-5,6-dihydro-4H-2,3,5,10b-tetraaza-benzo[e]azulene trihydrochloride;
8-Chloro-5-isopropyl-1-(3,4,5,6-tetrahydro-2H-[1,2']bipyridinyl-4-yl)-5,6-dihydro-4H-2,3,5,10b-tetraaza-benzo[e]azulene trihydrochloride;
8-Chloro-1-(3,4,5,6-tetrahydro-2H-[1,2']bipyridinyl-4-yl)-5-(tetrahydro-pyran-4-yl)-5,6-dihydro-4H-2,3,5,10b-tetraaza-benzo[e]azulene;
1-[8-Chloro-1-(3,4,5,6-tetrahydro-2H-[1,2']bipyridinyl-4-yl)-4H,6H-2,3,5,10b-tetraaza-benzo[e]azulen-5-yl]-ethanone dihydrochloride;
[8-Chloro-1-(3,4,5,6-tetrahydro-2H-[1,2']bipyridinyl-4-yl)-4H,6H-2,3,5,10b-tetraaza-benzo[e]azulen-5-yl]-cyclopropyl-methanone dihydrochloride;
1-[8-Chloro-1-(3,4,5,6-tetrahydro-2H-[1,2']bipyridinyl-4-yl)-4H,6H-2,3,5,10b-tetraaza-benzo[e]azulen-5-yl]-2,2-dimethyl-propan-1-one dihydrochloride;
8-Chloro-5-methanesulfonyl-1-(3,4,5,6-tetrahydro-2H-[1,2']bipyridinyl-4-yl)-5,6-dihydro-4H-2,3,5,10b-tetraaza-benzo[e]azulene;
8-Chloro-1-(1-pyrimidin-2-yl-piperidin-4-yl)-5,6-dihydro-4H-2,3,5,10b-tetraaza-benzo[e]azulene;
8-Chloro-5-methyl-1-(1-pyrimidin-2-yl-piperidin-4-yl)-5,6-dihydro-4H-2,3,5,10b-tetraaza-benzo[e]azulene;
8-Chloro-5-isopropyl-1-(1-pyrimidin-2-yl-piperidin-4-yl)-5,6-dihydro-4H-2,3,5,10b-tetraaza-benzo[e]azulene;
8-Chloro-5-methanesulfonyl-1-(1-pyrimidin-2-yl-piperidin-4-yl)-5,6-dihydro-4H-2,3,5,10b-tetraaza-benzo[e]azulene;
[8-Chloro-1- (1-pyrimidin-2-yl-piperidin-4-yl)-4H, 6H-2,3,5,10b-tetraaza-benzo[e]azulen-5-yl]-cyclopropyl-methanone;
1-[8-Chloro-1-(1-pyrimidin-2-yl-piperidin-4-yl)-4H,6H-2,3,5,10b-tetraaza-benzo[e]azulen-5-yl]-2,2-dimethyl-propan-1-one;
1-[8-Chloro-1-(1-pyrimidin-2-yl-piperidin-4-yl)-4H,6H-2,3,5,10b-tetraaza-benzo[e]azulen-5-yl]-ethanone;
8-Chloro-1-(6'-trifluoromethyl-3,4,5,6-tetrahydro-2H-[1,2']bipyridinyl-4-yl)-4H,6H-5-oxa-2,3,10b-triaza-benzo[e]azulene;
4-(8-Chloro-4H,6H-5-oxa-2,3,10b-triaza-benzo[e]azulen-1-yl)-3,4,5,6-tetrahydro-2H-[1,2']bipyridinyl-6'-carbonitrile;
4-(8-Chloro-4H,6H-5-oxa-2,3,10b-triaza-benzo[e]azulen-1-yl)-3,4,5,6-tetrahydro-2H-[1,2']bipyridinyl-6'-carboxylic acid amide;
13-Chloro-3-(3,4,5,6-tetrahydro-2H-[1,2']bipyridinyl-4-yl)-2,4,5,8-tetraaza-tricyclo[9.4.0.0*2,6*]pentadeca-1(11),3,5,12,14-pentaene;
1-[13-Chloro-3-(3,4,5,6-tetrahydro-2H-[1,2']bipyridinyl-4-yl)-2,4,5,8-tetraaza-tricyclo[9.4.0.0*2,6*]pentadeca-1(11),3,5,12,14-pentaen-8-yl]-ethanone;
13-Chloro-8-methyl-3-(3,4,5,6-tetrahydro-2H-[1,2']bipyridinyl-4-yl)-2,4,5,8-tetraaza-tricyclo[9.4.0.0*2,6*]pentadeca-1(11),3,5,12,14-pentaene;
3-(1-Pyrimidin-2-yl-piperidin-4-yl)-8-oxa-2,4,5-triaza-tricyclo[9.4.0.0*2,6*]pentadeca-1(11),3,5,12,14-pentaene;
8-Chloro-1-(1-pyrimidin-2-yl-piperidin-4-yl)-4H,6H-5-oxa-2,3,10b-triaza-benzo[e]azulene;
13-Chloro-3-(3,4,5,6-tetrahydro-2H-[1,2']bipyridinyl-4-yl)-8-oxa-2,4,5-triaza-tricyclo[9.4.0.0*2,6*]pentadeca-1(11),3,5,12,14-pentaene;
3-(3,4,5,6-Tetrahydro-2H-[1,2']bipyridinyl-4-yl)-8-oxa-2,4,5-triaza-tricyclo[9.4.0.0*2,6*]pentadeca-1(11),3,5,12,14-pentaene dihydrochloride;
8-Chloro-1-(3,4,5,6-tetrahydro-2H-[1,2']bipyridinyl-4-yl)-4H,6H-5-oxa-2,3,10b-triaza-benzo[e]azulene;
7-Chloro-1-(3,4,5,6-tetrahydro-2H-[1,2']bipyridinyl-4-yl)-4H,6H-5-oxa-2,3,10b-triaza-benzo[e]azulene dihydrochloride;
1-(3,4,5,6-Tetrahydro-2H-[1,2']bipyridinyl-4-yl)-4H,6H-5-oxa-2,3,10b-triaza-benzo[e]azulene;
8-Methoxy-1-(3,4,5,6-tetrahydro-2H-[1,2']bipyridinyl-4-yl)-4H,6H-5-oxa-2,3,10b-triaza-benzo[e]azulene dihydrochloride;
8-Fluoro-1-(3,4,5,6-tetrahydro-2H-[1,2']bipyridinyl-4-yl)-4H,6H-5-oxa-2,3,10b-triaza-benzo[e]azulene;
8,9-Difluoro-1-(3,4,5,6-tetrahydro-2H-[1,2']bipyridinyl-4-yl)-4H,6H-5-oxa-2,3,10b-triaza-benzo[e]azulene dihydrochloride;
9-Chloro-1-(3,4,5,6-tetrahydro-2H-[1,2']bipyridinyl-4-yl)-4H,6H-5-oxa-2,3,10b-triaza-benzo[e]azulene dihydrochloride;
1-(3,4,5,6-Tetrahydro-2H-[1,2']bipyridinyl-4-yl)-8-trifluoromethoxy-4H,6H-5-oxa-2,3,10b-triaza-benzo[e]azulene;
8-Methyl-1-(3,4,5,6-tetrahydro-2H-[1,2']bipyridinyl-4-yl)-4H,6H-5-oxa-2,3,10b-triaza-benzo[e]azulene; and
1-[8-Chloro-1-(3,4,5,6-tetrahydro-2H-[1,2']bipyridinyl-4-yl)-4H,6H-2,3,5,10b-tetraaza-benzo[e]azulen-5-yl]-2-dimethylamino-ethanone;
and pharmaceutically acceptable salts thereof.

5. The use according to claim 3, wherein the compound of formula (I) is 8-Chloro-5-methyl-1-(3,4,5,6-tetrahydro-2H-[1,2']bipyridinyl-4-yl)-5,6-dihydro-4H-2,3,5,10b-tetraazo-benzo[e]azulene, 8-Chloro-5-methyl-1-(1-pyrimidin-2-yl-piperidin-4-yl)-5,6-dihydro-4H-2,3,5,10b-tetraaza-benzo[e]azulene, or a pharmaceutically acceptable salt thereof.

6. Use of a vasopressin V1a receptor antagonist in the manufacture of a medicament for the treatment of premature ejaculation or rapid ejaculation.

7. The use of claim 6, wherein the IC₅₀ of the vasopressin V1a receptor antagonist is less than 100nM.

8. The use of claim 7, wherein the vasopressin V1a receptor antagonist is selective for vasopressin V1a receptors.

9. A method of screening for compounds useful for the treatment of premature ejaculation or rapid ejaculation, comprising screening compounds for antagonist activity against vasopressin V1a receptors, and selecting compounds with an IC₅₀ of less than 100 nM.

10. Use of a combination of a vasopressin V1 a receptor antagonist with a PDE V inhibitor for the preparation of a medicament for the treatment of premature ejaculation or rapid ejaculation.

11. Use of a combination of a vasopressin V1a receptor antagonist with a selective serotonin reuptake inhibitor (SSRI) for the preparation of a medicament for the treatment of premature ejaculation or rapid ejaculation.

12. Products containing a vasopressin V1a receptor antagonist and a PDE V inhibitor as a combined preparation for simultaneous, separate or sequential use in treating premature ejaculation or rapid ejaculation.

13. Products containing a vasopressin V1a receptor antagonist and a selective serotonin reuptake inhibitor (SSRI) for the preparation of a medicament for the treatment of premature ejaculation or rapid ejaculation.

## Patentansprüche

1. Verwendung einer Verbindung der Formel (Ia): 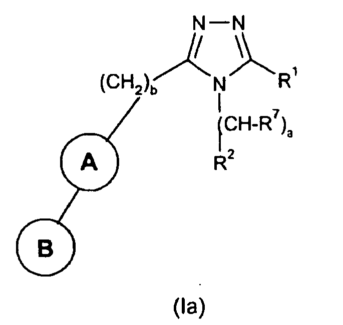 oder eines pharmazeutisch verträglichen Salzes oder Solvats davon, worin
R¹ C₁-C₆-Alkyl, -(CH₂)_{c}[C₃-C₈-Cycloalkyl]-, -(CH₂)_{c}-W oder -(CH₂)_{c}-Z-(CH₂)_{d}-W wiedergibt;
R² eine Phenylgruppe wiedergibt, gegebenenfalls kondensiert mit einer 5- oder 6-gliedrigen Aryl- oder heterocyclischen Gruppe, die ein oder mehrere Heteroatome, ausgewählt aus N, 0 oder S, enthalten kann; wobei die Phenylgruppe und die gegebenenfalls kondensierte Gruppe gegebenenfalls mit einer oder mehreren Gruppen, unabhängig ausgewählt aus der nachstehend definierten Liste, substituiert sind;
Ring **A** eine 4-, 5- oder 6-gliedrige gesättigte, heterocyclische Gruppe, die mindestens ein N enthält, wiedergibt;
Ring **B** eine Phenylgruppe oder Het¹ wiedergibt, wobei jede Gruppe gegebenenfalls mit einer oder mehreren Gruppen, unabhängig ausgewählt aus der nachstehend definierten Liste, substituiert ist;
Het¹ eine 4-, 5- oder 6-gliedrige gesättigte oder ungesättigte, heterocyclische Gruppe, die mindestens ein N enthält (die jedoch auch ein oder mehrere O- oder S-Atome enthalten kann), wiedergibt;
R⁷ unabhängig H, C₁-C₆-Alkyl, OR³, -(CH₂)ₑ-R³ oder -(CH₂)_{f}-O-(CH₂)ₑ-R³ wiedergibt;
W eine Phenylgruppe, NR⁴R⁵ oder Het² wiedergibt, wobei die Phenylgruppe gegebenenfalls mit einer oder mehreren Gruppen, unabhängig ausgewählt aus Halogen, CF₃, OCF₃, R³, OR³, CO₂R³, CONR⁴R⁵, CN, SO₂NR⁴R⁵ und NR³SO₂Me, substituiert ist;
Het² eine 4-, 5-, 6- oder 7-gliedrige gesättigte oder ungesättigte, heterocyclische Gruppe, die mindestens ein N enthält (die jedoch auch ein oder mehrere O- oder S-Atome enthalten kann), gegebenenfalls substituiert mit einer oder mehreren Gruppen, unabhängig ausgewählt aus der nachstehend definierten Liste, wiedergibt;
Z O oder S(O)_{g} wiedergibt;
g 0, 1 oder 2 wiedergibt;
Het³ eine 4-, 5-, 6- oder 7-gliedrige gesättigte oder ungesättigte, heterocyclische Gruppe, die mindestens ein N enthält (die jedoch auch ein oder mehrere O- oder S-Atome enthalten kann), gegebenenfalls substituiert mit einer oder mehreren Gruppen, unabhängig ausgewählt aus der nachstehend definierten Liste, wiedergibt;
bei jedem Auftreten, R³ und R⁶ unabhängig H, C₁-C₆-Alkyl , gegebenenfalls substituiert mit Y, -(CH₂)_{g}-[C₃-C₈-Cycloalkyl], Phenyl, Benzyl, Pyridyl oder Pyrimidyl, wiedergeben; Y unabhängig eine Phenylgruppe, NR⁴R⁵ oder Het³ wiedergibt, wobei die Phenylgruppe gegebenenfalls mit einer oder mehreren Gruppen, unabhängig ausgewählt aus Halogen, CF₃, OCF₃, R⁴, OR ⁴ CO₂R⁴, CONR⁴R⁵, CN, SO₂NR⁴R⁵, NR⁴SO₂Me und -NR⁴R⁵, substituiert ist;
bei jedem Auftreten, R⁴ und R⁵ unabhängig H, C₁-C₆-Alkyl, -(CH₂)_{g}-[C₃-C₈-Cycloalkyl], Phenyl, Benzyl, Pyridyl oder Pyrimidyl wiedergeben; oder R⁴ und R⁵, zusammen mit dem N-Atom, an das sie gebunden sind, eine heterocyclische Gruppe mit 3 bis 8 Atomen wiedergeben;
Substituenten für R², Ring **B,** Het¹, Het² und Het³ unabhängig ausgewählt sind aus der nachstehenden Liste: Halogen, CF₃, OCF₃, R³, -(CH₂)ₑOR³, -(CH₂)ₑ-CO₂R³, -(CH₂)ₑ-CONR⁴R⁵, -(CH₂)ₑ-CN, -(CH₂)ₑ-SO₂NR⁴R⁵, -(CH₂)ₑ-NR³SO₂Me, -(CH₂)ₑ-COR³, -(CH₂)ₑ-OCOR³, -(CH₂)ₑ-NHCOR³, -(CH₂)ₑ-NR³COR⁶ und -(CH₂)ₑNR⁴R⁵;
a und b unabhängig 0 oder 1 wiedergeben;
c, d, e und g unabhängig 0, 1, 2, 3 oder 4 wiedergeben;
f unabhängig 1, 2, 3 oder 4 wiedergibt;
mit der Maßgabe, dass a + b nicht gleich 0 sein kann; und mit der Maßgabe, dass, wenn R¹-(CH₂)_{c}-Z-(CH₂)_{d}-W wiedergibt und W NR⁴R⁵ oder eine beliebige N-verknüpfte heterocyclische Gruppe wiedergibt, dann d nicht 0 oder 1 sein darf; und
mit der Maßgabe, dass, wenn R² eine Phenylgruppe, substituiert mit einer Gruppe der Formel -(CH₂)ₑOR³, -(CH₂)ₑ-CO₂R³ oder -(CH₂)ₑOCOR³, wiedergibt; oder
Het¹ und/oder Het² mit einer Gruppe der Formel -(CH₂)ₑOR³, -(CH₂)ₑ-CO₂R³ oder -(CH₂)ₑOCOR³ substituiert sind; oder
wenn R⁷ -OR³ oder -(CH₂)_{f}-O-(CH₂)ₑ-R³ wiedergibt und e 0 ist; oder
wenn W eine Phenylgruppe, substituiert mit -OR³ oder -CO₂R³, wiedergibt;
und _{R}³ eine Alkylgruppe, substituiert mit Y, wiedergibt und Y NR⁴R⁵ oder ein N-verknüpftes Het³ wiedergibt;
dann R³ C₂-C₆-Alkyl, substituiert mit Y, wiedergeben muss, bei der Herstellung eines Medikaments für die Behandlung frühzeitiger Ejakulation.

2. Verwendung nach Anspruch 1, wobei die Verbindung der Formel (Ia) 4-[4-Benzyl-5-(4-methoxy-piperidin-1-ylmethyl)-4H-[1,2,4]triazol-3-yl]-3,4,5, 6-tetrahydro-2H-[1,2']bipyridinyl oder ein pharmazeutisch verträgliches Salz davon darstellt.

3. Verwendung einer Verbindung der Formel (I): 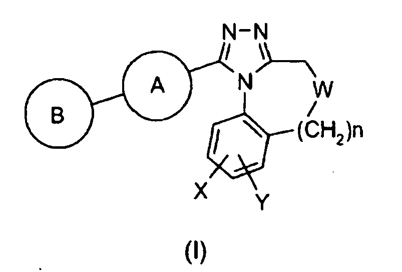 oder eines pharmazeutisch verträglichen Salzes oder Solvats davon, worin
W O, S oder NR¹ darstellt;
R¹ H, C₁₋₆-Alkyl, -(CH₂)ₐ-[C₃₋₈-Cycloalkyl], Phenyl, Benzyl, Pyridyl, Pyrimidyl, -COR², -CO₂R², -CO-(CH₂)ₐ-NR²R³, -SO₂R², -(CH₂)_{b}-OR², -(CH₂)_{b}-NR²R³, oder einen gesättigten Heterocyclus mit 3 bis 8 Atomen, der ein oder mehrere Heteroatom(e), ausgewählt aus O, N und S, enthält, wiedergibt;
X und Y unabhängig H, Halogen, OH, CF₃, OCF₃, R⁴, -(CH₂)_{d}-CONR⁴R⁵, -(CH₂)_{d}-CN, -(CH₂)_{d}-SO₂NR⁴R⁵, -(CH₂)_{d}-NR⁴SO₂Me, -(CH₂)_{d}-COR⁴, -(CH₂)_{d}-OCOR⁴, -(CH₂)_{d}-NHCOR⁴, -(CH₂)_{d}-NR⁴COR⁵, -(CH₂)_{d}-OR⁶ oder -(CH₂)_{d}-CO₂R⁶ wiedergeben;
Ring **A** eine Piperidinyl-, Piperazinyl-, Pyrrolidinyl- oder Azetidinylgruppe wiedergibt;
Ring **B** eine Phenyl-, Pyridinyl- oder Pyrimidinylgruppe wiedergibt (gegebenenfalls substituiert mit einer oder mehreren Gruppen, unabhängig ausgewählt aus Halogen, CN, CONH₂, CF₃, OCF₃, R⁷ und -(CH₂)_{f}-OR⁸);
R², R³, R⁴, R⁵ und R⁷ unabhängig H, gerades oder verzweigtes C₁₋₆-Alkyl, -(CH₂)_{c}-[C₃₋₈-Cycloalkyl], Phenyl, Benzyl, Pyridyl oder Pyrimidyl wiedergeben;
oder R² und R³, oder R⁴ und R⁵, zusammen mit dem Stickstoffatom, an das sie gebunden sind, unabhängig einen Heterocyclus mit 3 bis 8 Atomen wiedergeben;
R⁶ und R⁸ unabhängig H, gerades oder verzweigtes C₁-₆-Alkyl, -(CH₂)_{c}-[C₃₋₈-Cycloalkyl], -(CH₂)ₑ-NR⁴R⁵, -(CH₂)ₑ-OR⁴, Phenyl, Benzyl, Pyridyl oder Pyrimidyl wiedergeben;
n = 0, 1 oder 2;
a, c, d und f jeweils unabhängig ausgewählt sind aus 0, 1, 2 und 3;
b und e jeweils unabhängig ausgewählt sind aus 2 und 3, bei der Herstellung eines Medikaments für die Behandlung von frühzeitiger Ejakulation oder schneller Ejakulation.

4. Verwendung nach Anspruch 3, wobei die Verbindung der Formel (I) ausgewählt ist aus:
1-(3,4,5,6-Tetrahydro-2H-[1,2']bipyridinyl-4-yl)-5,6-dihydro-4H-2,3,5,10b-tetraaza-benzo[e]azulen;
5-Methyl-1-(3,4,5,6-tetrahydro-2H-[1,2']bipyridinyl-4-yl)-5,6-dihydro-4H-2,3,5,10b-tetraaza-benzo[e]azulen;
1-[1-(3,4,5,6-Tetrahydro-2H-[1,2']bipyridinyl-4-yl)-4H,6H-2,3,5,10b-tetraaza-benzo[e]azulen-5-yl]-ethanon;
8-Chlor-1-(3,4,5,6-tetrahydro-2H-[1,2']bipyridinyl-4-yl)-5,6-dihydro-4H-2,3,5,10b-tetraaza-benzo[e]azulen;
8-Chlor-5-methyl-1-(3,4,5,6-tetrahydro-2H-[1,2']bipyridinyl-4-yl)-5,6-dihydro-4H-2,3,5,10b-tetraaza-benzo[e]azulentrihydrochlorid;
8-Chlor-5-isopropyl-1-(3,4,5,6-tetrahydro-2H-[1,2']bipyridinyl-4-yl)-5,6-dihydro-4H-2,3,5,10b-tetraaza-benzo[e]azulentrihydrochlorid;
8-Chlor-1-(3,4,5,6-tetrahydro-2H-[1,2']bipyridinyl-4-yl)-5-(tetrahydro-pyran-4-yl)-5,6-dihydro-4H-2,3,5,10b-tetraaza-benzo[e]azulen;
1-[8-Chlor-1-(3,4,5,6-tetrahydro-2H-[1,2']bipyridinyl-4-yl)-4H, 6H-2,3,5,10b-tetraaza-benzo[e]azulen-5-yl]-ethanondihydrochlorid;
[8-Chlor-1-(3,4,5,6-tetrahydro-2H-[1,2']bipyridinyl-4-yl)-4H, 6H-2,3,5,10b-tetraaza-benzo[e]azulen-5-yl]-cyclopropylmethanondihydrochlorid;
1-[8-Chlor-1-(3,4,5,6-tetrahydro-2H-[1,2']bipyridinyl-4-yl)-4H, 6H-2,3,5,10b-tetraaza-benzo [e]azulen-5-yl]-2,2-dimethylpropan-1-ondihydrochlorid;
8-Chlor-5-methansulfonyl-1-(3,4,5,6-tetrahydro-2H-[1,2']bipyridin-yl-4-yl)-5,6-dihydro-4H-2,3,5,10b-tetraaza-benzo[e]-azulen;
8-Chlor-1-(1-pyrimidin-2-yl-piperidin-4-yl)-5,6-dihydro-4H-2,3,5,10b-tetraaza-benzo[e]azulen;
8-Chlor-5-methyl-1-(1-pyrimidin-2-yl-piperidin-4-yl)-5,6-dihydro-4H-2,3,5,10b-tetraaza-benzo[e]azulen;
8-Chlor-5-isopropyl-1-(1-pyrimidin-2-yl-piperidin-4-yl)-5,6-dihydro-4H-2,3,5,10b-tetraaza-benzo[e]azulen;
8-Chlor-5-methansulfonyl-1-(1-pyrimidin-2-yl-piperidin-4-yl)-5,6-dihydro-4H-2,3,5,10b-tetraaza-benzo[e]azulen;
[8-Chlor-1-(1-pyrimidin-2-yl-piperidin-4-yl)-4H, 6H-2 , 3 , 5 , 10b-tetraaza-benzo[e]azulen-5-yl]-cyclopropyl-methanon;
1-[8-Chlor-1-(1-pyrimidin-2-yl-piperidin-4-yl)-4H, 6H-2,3,5,10b-tetraaza-benzo[e]azulen-5-yl]-2,2-dimethyl-propan-1-on;
1-[8-Chlor-1-(1-pyrimidin-2-yl-piperidin-4-yl)-4H,6H-2,3,5,10b-tetraaza-benzo[e]azulen-5-yl]-ethanon;
8-Chlor-1-(6'-trifluormethyl-3,4,5,6-tetrahydro-2H-[1,2']bipyridinyl-4-yl)-4H,6H-5-oxa-2,3,10b-triaza-benzo[e]azulen;
4-(8-Chlor-4H,6H-5-oxa-2,3,10b-triaza-benzo[e]azulen-1-yl)-3,4,5,6-tetrahydro-2H-[1,2']bipyridinyl-6'-carbonitril;
4-(8-Chlor-4H, 6H-5-oxa-2,3,10b-triaza-benzo [e]azulen-1-yl)-3,4,5,6-tetrahydro-2H-[1,2']bipyridinyl-6'-carbonsäureamid;
13-Chlor-3-(3,4,5,6-tetrahydro-2H-[1,2']bipyridinyl-4-yl)-2,4,5,8-tetraaza-tricyclo[9.4.0.0*2,6*]pentadeca-1(11),3,5,12,14-pentaen;
1-[13-Chlor-3-(3,4,5,6-tetrahydro-2H-[1,2']bipyridinyl-4-yl)-2,4,5,8-tetraaza-tricyclo[9.4.0.0*2, 6*]pentadeca-1(11),3,5,12,14-pentaen-8-yl]-ethanon;
13-Chlor-8-methyl-3-(3,4,5,6-tetrahydro-2H-[1,2'] bipyridinyl-4-yl)-2,4,5,8-tetraaza-tricyclo[9.4.0.0*2,6*]pentadeca-1(11),3,5,12,14-pentaen;
3-(1-Pyrimidin-2-yl-piperidin-4-yl)-8-oxa-2,4,5-triaza-tricyclo[9.4.0.0*2,6*]pentadeca-1(11),3,5,12,14-pentaen;
8-Chlor-1-(1-pyrimidin-2-yl-piperidin-4-yl)-4H,6H-5-oxa-2,3,10b-triaza-benzo[e]azulen;
13-Chlor-3-(3,4,5,6-tetrahydro-2H-[1,2']bipyridinyl-4-yl)-8-oxa-2,4,5-triaza-tricylo[9.4.0.0*2,6*]pentadeca-1(11),3,5,12,14-pentaen;
3-(3,4,5,6-Tetrahydro-2H-[1,2']bipyridinyl-4-yl)-8-oxa-2,4,5-triaza-tricyclo[9.4.0.0*2,6*]pentadeca-1(11),3,5,12,14-pentaendihydrochlorid;
8-Chlor-1-(3,4,5,6-tetrahydro-2H-[1,2']bipyridinyl-4-yl)-4H,6H-5-oxa-2,3,10b-triaza-benzo[e]azulen;
7-Chlor-1-(3,4,5, 6-tetrahydro-2H-[1,2'] bipyridinyl-4-yl)-4H,6H-5-oxa-2,3,10b-triaza-benzo[e]azulendihydrochlorid;
1-(3,4,5,6-Tetrahydro-2H-[1,2']bipyridinyl-4-yl)-4H,6H-5-oxa-2,3,10b-triaza-benzo[e]azulen;
8-Methoxy-1-(3,4,5,6-tetrahydro-2H-[1,2']bipyridinyl-4-yl)-4H,6H-5-oxa-2,3,10b-triaza-benzo[e]azulendihydrochlorid;
8-Fluor-1-(3,4,5,6-tetrahydro-2H-[1,2']bipyridinyl-4-yl)-4H,6H-5-oxa-2,3,10b-triaza-benzo[e]azulen;
8,9-Difluor-1-(3,4,5,6-tetrahydro-2H-[1,2']bipyridinyl-4-yl)-4H,6H-5-oxa-2,3,10b-triaza-benzo[e]azulendihydrochlorid;
9-Chlor-1-(3,4,5,6-tetrahydro-2H-[1,2']bipyridinyl-4-yl)-4H, 6H-5-oxa-2,3,10b-triaza-benzo[e]azulendihydrochlorid;
1-(3, 4, 5, 6-Tetrahydro-2H-[1,2']bipyridinyl-4-yl)-8-trifluormethoxy-4H, 6H-5-oxa-2,3,10b-triaza-benzo[e]azulen;
8-Methyl-1-(3,4,5,6-tetrahydro-2H-[1,2']bipyridinyl-4-yl)-4H,6H-5-oxa-2,3,10b-triaza-benzo[e]azulen und
1-[8-Chlor-1-(3,4,5,6-tetrahydro-2H-[1,2']bipyridinyl-4-yl)-4H,6H-2,3,5,10b-tetraaza-benzo[e]azulen-5-yl]-2-dimethylamino-ethanon; und den pharmazeutisch verträglichen Salzen davon.

5. Verwendung nach Anspruch 3, wobei die Verbindung der Formel (I) 8-Chlor-5-methyl-1-(3,4,5,6-tetrahydro-2H-[1,2']bipyridinyl-4-yl)-5,6-dihydro-4H-2,3,5,10b-tetraazo-benzo[e]-azulen, 8-Chlor-5-methyl-1-(1-pyrimidin-2-yl-piperidin-4-yl)-5,6-dihydro-4H-2,3,5,10b-tetraaza-benzo[e]azulen oder ein pharmazeutisch verträgliches Salz davon darstellt.

6. Verwendung eines Vasopressin-V1a-Rezeptor-Antagonisten bei der Herstellung eines Medikaments für die Behandlung von frühzeitiger Ejakulation oder schneller Ejakulation.

7. Verwendung nach Anspruch 6, wobei der IC₅₀ von dem Vasopressin-V1a-Rezeptor-Antagonisten weniger als 100 nM ist.

8. Verwendung nach Anspruch 7, wobei der Vasopressin-V1a-Rezeptor-Antagonist für Vasopressin-V1a-Rezeptoren selektiv ist.

9. Verfahren zum Absuchen bzw. Screening nach Verbindungen, die bei der Behandlung von frühzeitiger Ejakulation oder schneller Ejakulation nützlich sind, umfassend Absuchen nach Verbindungen hinsichtlich einer Antagonistenwirksamkeit gegen Vasopressin-V1a-Rezeptoren, und Auswählen von Verbindungen mit einem IC₅₀ von weniger als 100 nM.

10. Verwendung einer Kombination von einem Vasopressin-V1a-Rezeptor-Antagonisten mit einem PDE-V-Inhibitor zur Herstellung eines Medikaments für die Behandlung von frühzeitiger Ejakulation oder schneller Ejakulation.

11. Verwendung einer Kombination von einem Vasopressin-V1a-Rezeptor-Antagonisten mit einem selektiven Serotonin-Reuptake-Inhibitor (SSRI) für die Herstellung eines Medikaments für die Behandlung von frühzeitiger Ejakulation oder schneller Ejakulation.

12. Produkte, enthaltend einen Vasopressin-V1a-Rezeptor-Antagonisten und einen PDE-V-Inhibitor als eine kombinierte Zubereitung für gleichzeitige, getrennte oder aufeinander folgende Anwendung bei der Behandlung von frühzeitiger Ejakulation oder schneller Ejakulation.

13. Produkte, die einen Vasopressin-V1a-Rezeptor-Antagonisten und einen selektiven Serotonin-Reuptake-Inhibitor (SSRI) enthalten, für die Herstellung eines Medikaments für die Behandlung von frühzeitiger Ejakulation oder schneller Ejakulation.

## Revendications

1. Utilisation d'un composé de formule (la) : 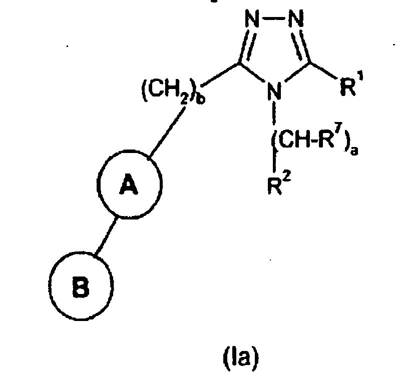 ou d'un de ses sels ou produits de solvatation pharmaceutiquement acceptables, formule dans laquelle :
R¹ représente un groupe alkyle en C₁ à C₆, -(CH₂)_{c}-[cycloalkyle en C₃ à C₈], -(CH₂)_{c}-W ou -(CH₂)_{c}-Z-(CH₂)_{d}-W ;
R² représente un groupe phényle, facultativement condensé à un groupe aryle ou hétérocyclique penta- ou hexagonal qui peut contenir un ou plusieurs hétéroatomes choisis entre N, O et S ; le groupe phényle et le groupe facultativement condensé étant facultativement substitués avec un ou plusieurs groupes choisis indépendamment dans la liste définie ci-dessous ;
le noyau A représente un groupe hétérocyclique saturé tétra-, penta- ou hexagonal contenant au moins un atome de N ;
le noyau B représente un groupe phényle ou het¹, chaque groupe étant facultativement substitué avec un ou plusieurs groupes choisis indépendamment dans la liste définie ci-dessous ;
het¹ représente un groupe hétérocyclique tétra-, penta- ou hexagonal, saturé ou insaturé, contenant au moins un atome de N (mais qui peut contenir également un ou plusieurs atomes de O ou S) ;
R⁷ représente indépendamment H, un groupe alkyle en C₁ à C₆, OR³, -(CH₂)ₑ-R³ ou -(CH₂)_{f}-O-(CH₂)ₑ-R³ ;
W représente un groupe phényle, NR⁴R⁵ ou het², le groupe phényle étant facultativement substitué avec un ou plusieurs groupes choisis indépendamment entre des groupes halogéno, CF₃, OCF₃, R³, OR³, CO₂R³, CONR⁴R⁵, CN, SO₂NR⁴R⁵ et NR³SO₂Me ;
het² représente un groupe hétérocyclique tétra-, penta-, hexa- ou heptagonal, saturé ou insaturé, contenant au moins un atome de N (mais qui peut contenir également un ou plusieurs atomes de O ou S), facultativement substitué avec un ou plusieurs groupes choisis indépendamment dans la liste définie ci-dessous ;
Z représente 0 ou un groupe S(O)g ;
g est égal à 0, 1 ou 2 ;
het³ représente un groupe hétérocyclique tétra-, penta-, hexa- ou heptagonal, saturé ou insaturé, contenant au moins un atome de N (mais qui peut contenir également un ou plusieurs atomes de O ou S), facultativement substitué avec un ou plusieurs groupes choisis indépendamment dans la liste définie ci-dessous ;
à chaque occurrence, R³ et R⁶ représentent indépendamment H, un groupe alkyle en C₁ à C₆ facultativement substitué avec un substituant Y, -(CH₂)_{g}-[cycloalkyle en C₃ à C₈], phényle, benzyle, pyridyle ou pyrimidyle ;
Y représente indépendamment un groupe phényle, NR⁴R⁵ ou het³, le groupe phényle étant facultativement substitué avec un ou plusieurs groupes choisis indépendamment entre des groupes halogéno, CF₃, OCF₃, R⁴, OR⁴, CO₂R⁴, CONR⁴R⁵, CN, SO₂NR⁴R⁵, NR⁴SO₂Me et -NR⁴R⁵ ;
à chaque occurrence, R³ et R⁵ représentent indépendamment H, un groupe alkyle en C₁ à C₆, -(CH₂)_{g}-[cycloalkyle en C₃ à C₈], phényle, benzyle, pyridyle ou pyrimidyle ; ou bien R⁴ et R⁵, conjointement avec l'atome de N auquel ils sont fixés, représentent un groupe hétérocyclique de 3 à 8 atomes ;
les substituants pour R², le noyau B, het¹, het² et het³ sont choisis indépendamment dans la liste suivante : halogéno, CF₃, OCF₃, R³, -(CH₂)ₑ-OR³, -(CH₂)ₑ-CO₂R³, -(CH₂)ₑ-CONR⁴R⁵, -(CH²)ₑ-CN, -(CH₂)ₑ-SO₂NR⁴R⁵, -(CH₂)ₑ-NR³SO₂Me, - (CH₂)ₑ-COR³, -(CH₂)ₑ-OCOR³, -(CH₂)ₑ-NHCOR³, -(CH₂)ₑ-NR³COR⁶ et -(CH₂)ₑNR⁴R⁵ ;
a et b sont égaux indépendamment à 0 ou 1 ;
c, d, e et g sont égaux indépendamment à 0, 1, 2, 3 ou 4 ;
f est égal indépendamment à 1, 2, 3 ou 4 ;
sous réserve que a + b ne puisse être égal à 0 ; et
sous réserve que, lorsque R¹ représente un groupe - (CH₂)_{c}-Z- (CH₂)_{d}-W et W représente un groupe NR⁴R⁵, ou n'importe quel groupe hétérocyclique lié par N, alors d ne doit pas être égal à 0 ou 1 ; et
sous réserve que, lorsque R² représente un groupe phényle substitué avec un groupe de formule -(CH₂)ₑOR³, -(CH₂)ₑ-CO₂R³ ou -(CH₂)ₑOCOR³ ; ou
het¹ et/ou het² sont substitués avec un groupe de formule -(CH₂)ₑOR³, -(CH₂)ₑCO₂R³, ou -(CH₂)ₑOCOR³ ; ou
lorsque R⁷ représente un groupe -OR³ ou -(CH₂)_{f}-O-(CH₂) ₑ-R³ et e est égal à 0 ; ou
lorsque W représente un groupe phényle substitué avec un substituant -OR³ ou -CO₂R³ ;
et R³ représente un groupe alkyle substitué avec Y, et Y représente un groupe NR⁴R⁵ ou un groupe het³ lié par N ;
alors R³ doit représenter un groupe alkyle en C₂ à C₆ substitué avec Y,
dans la production d'un médicament destiné au traitement de l'éjaculation précoce.

2. Utilisation suivant la revendication 1, dans laquelle le composé de formule (Ia) est le 4-[4-benzyl-5-(4-méthoxy-pipéridine-1-ylméthyl)-4H-[1,2,4]triazole-3-yl]-3,4,5,6-tétrahydro-2H-[1,2']bipyridinyle ou un de ses sels pharmaceutiquement acceptables.

3. Utilisation d'un composé de formule (I) : 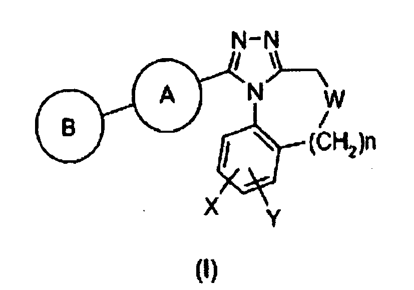 ou d'un de ses sels ou produits de solvatation pharmaceutiquement acceptables, formule dans laquelle
W représente O, S ou un groupe NR¹ ;
R¹ représente H, un groupe alkyle en C₁ à C₆, - (CH₂)ₐ-[cycloalkyle en C₃ à C₈], phényle, benzyle, pyridylé, pyrimidyle, -COR², -CO₂R², -CO-(CH₂)ₐ-NR²R³, -SO₂R², -(CH₂)_{b}-OR², -(CH₂)_{b}-NR²R³ ou un hétérocycle saturé de 3 à 8 atomes contenant un ou plusieurs hétéroatomes choisis entre O, N et S ;
X et Y représentent indépendamment H, un groupe halogéno, OH, CF₃, OCF₃, R⁴, -(CH₂)_{d}-CONR⁴R⁵, -(CH₂)_{d}-CN, -(CH₂)_{d}-SO₂NR⁴R⁵, -(CH₂)_{d}-NR⁴SO₂Me, -(CH₂)_{d}-COR⁴, -(CH₂)_{d}-OCOR₄, -(CH₂)_{d}-NHCOR⁴, -(CH₂)_{d}-NR⁴COR⁵, -(CH₂)_{d}-OR⁶ ou-(CH₂)_{d}-CO₂R⁶ ;
le noyau A représente un groupe pipéridinyle, pipérazinyle, pyrrolidinyle ou azétidinyle ;
le noyau B représente un groupe phényle, pyridinyle ou pyrimidinyle (facultativement substitué avec un ou plusieurs groupes choisis indépendamment entre des groupes halogéno, CN, CONH₂, CF₃, OCF₃, R⁷ et -(CH₂)_{f}-OR⁸) ;
R², R³, R⁴, R⁵ et R⁷ représentent indépendamment H, un groupe alkyle en C₁ à C₆ droit ou ramifié, -(CH₂)_{c}-[cycloalkyle en C₃ à C₈], phényle, benzyle, pyridyle ou pyrimidyle ;
ou bien R² et R³, ou R⁴ et R⁵, conjointement avec l'atome d'azote auquel ils sont fixés, représentent indépendamment un hétérocycle de 3 à 8 atomes ;
R⁶ et R⁸ représentent indépendamment H, un groupe alkyle en C₁ à C₆ droit ou ramifié, -(CH₂)_{c}-[cycloalkyle en C₃ à C₈], -(CH₂)ₑ-NR⁴R⁵, -(CH₂)ₑ-OR⁴, phényle, benzyle, pyridyle ou pyrimidyle ;
n est égal à 0, 1 ou 2 ;
a, c, d et f sont choisis chacun indépendamment entre 0, 1, 2 et 3 ;
b et e sont choisis chacun indépendamment entre 2 et 3 ;
dans la production d'un médicament destiné au traitement de l'éjaculation précoce ou de l'éjaculation rapide.

4. Utilisation suivant la revendication 3, dans laquelle le composé de formule (I) est choisi entre :
le 1-(3,4,5,6-tétrahydro-2H-[1,2']bipyridinyl-4-yl)-5,6-dihydro-4H-2,3,5,10b-tétraaza-benzo[e]azulène ;
le 5-méthyl-1-(3,4,5,6-tétrahydro-2H-[1,2']bipyridinyl-4-yl)-5,6-dihydro-4H-2,3,5,1Ob-tétraaza-benzo[e]azulène ;
la 1-[1-(3,4,5,6-tétrahydro-2H-[1,2']bipyridinyl-4-yl)-4H,6H-2,3,5,10b-tétraaza-benzo[e]azulène-5-yl]-éthanone ;
le 8-chloro-1-(3,4,5,6-tétrahydro-2H-[1,2']bipyridinyl-4-yl)-5,6-dihydro-4H-2,3,5,10b-tétraaza-benzo[e]azulène ;
le trichlorhydrate de 8-chloro-5-méthyl-1-(3,4,5,6-tétrahydro-2H-[1,2']bipyridinyl-4-yl)-5,6-dihydro-4H-2,3,5,10b-tétraaza-benzo[e]azulène ;
le trichlorhydrate de 8-chloro-5-isopropyl-1-(3,4,5,6-tétrahydro-2H-[1,2']bipyridinyl-4-yl)-5,6-dihydro-4H-2,3,5,10b-tétraaza-benzo[e]azulène ;
le 8-chloro-1-(3,4,5,6-tétrahydro-2H-[1,2']bipyridinyl-4-yl)-5-(tétrahydro-pyranne-4-yl)-5,6-dihydro-4H-2,3,5,10b-tétraaza-benzo[e]azulène ;
le dichlorhydrate de 1-[8-chloro-1-(3,4,5,6-tétrahydro-2H-[1,2']bipyridinyl-4-yl)-4H,6H-2,3,5,10b-tétraazabenzo[e]azulène-5-yl]-éthanone ;
le dichlorhydrate de [8-chloro-1-(3,4,5,6-tétrahydro-2H-[1,2']bipyridinyl-4-yl)-4H,6H-2,3,5,10b-tétraazabenzo[e]azulène-5-yl]-cyclopropyl-méthanone ;
le dichlorhydrate de 1-[8-chloro-1-(3,4,5,6-tétrahydro-2H-[1,2']bipyridinyl-4-yl)-4H,6H-2,3,5,10b-tétraazabenzo[e]azulène-5-yl]-2,2-diméthyl-propane-1-one ;
le 8-chloro-5-méthanesulfonyl-1-(3,9,5,6-tétrahydro-2H-[1,2']bipyridinyl-4-yl)-5,6-dihydro-4H-2,3,5,10b-tétraazabenzo[e]azulène ;
le 8-chloro-1-(1-pyrimidine-2-yl-pipéridine-4-yl)-5,6-dihydro-4H-2,3,5,10b-tétraaza-benzo[e]azulène ;
le 8-chloro-5-méthyl-1-(1-pyrimidine-2-yl-pipéridine-4-yl)-5,6-dihydro-4H-2,3,5,10b-tétraaza-benzo[e]azulène ;
le 8-chloro-5-isopropyl-1-(1-pyrimidine-2-yl-pipéridine-4-yl)-5,6-dihydro-4H-2,3,5,10b-tétraaza-benzo[e]azulène ;
le 8-chloro-5-méthanesulfonyl-1-(1-pyrimidine-2-yl-pipéridine-4-yl)-5,6-dihydro-4H-2,3,5,10b-tétraaza-benzo[e]azulène ;
la [8-chloro-1-(1-pyrimidine-2-yl-pipéridine-4-yl)-4H,6H-2,3,5,10b-tétraaza-benzo[e]azulène-5-yl]-cyclopropyl-méthanone ;
la 1-[8-chloro-1-(1-pyrimidine-2-yl-pipéridine-4-yl)-4H,6H-2,3,5,10b-tétraaza-benzo[e]azulène-5-yl]-2,2-diméthyl-propane-1-one ;
la 1- [8-chloro-1-(1-pyrimidine-2-yl-pipéridine-4-yl)-4H,6H-2,3,5,10b-tétraaza-benzo[e]azulène-5-yl]-éthanone ;
le 8-chloro-1-(6'-trifluorométhyl-3,4,5,6-tétrahydro-2H-[1,2']bipyridinyl-4-yl)-4H,6H-5-oxa-2,3,10b-triaza-benzo[e]-azulène ;
le 4-(8-chloro-4H,6H-5-oxa-2,3,10b-triaza-benzo[e]azulène-1-yl)-3,4,5,6-tétrahydro-2H-[1,2']bipyridinyl-6'-carbonitrile ;
l'amide d'acide 4-(8-chloro-4H,6H-5-oxa-2,3,10b-triazabenzo[e]azulène-1-yl)-3,4,5,6-tétrahydro-2H-[1,2']bipyridinyl-6'-carboxylique :
le 13-chloro-3-(3,4,5,6-tétrahydro-2H-[1,2']bipyridinyl-4-yl)-2,4,5,8-tétraaza-tricyclo[9.4.0.0^{*}2,6^{*}]pentadéca-1(11),3,5,12,14-pentaène ;
la 1-[13-chloro-3-(3,4,5,6-tétrahydro-2H-[1,2']bipyridinyl-4-yl)-2,4,5,8-tétraaza-tricyclo[9.4.0.0*2,6*]pentadéca-1(11),3,5,12,14-pentaène-8-yl]-éthanone ;
le 13-chloro-8-méthyl-3-(3,4,5,6-tétrahydro-2H-[1,2']-bipyridinyl-4-yl)-2,4,5,8-tétraaza-tricyclo[9.4.0.0*2,6*]-pentadéca-1(11),3,5,12,14-pentaène ;
le 3-(1-pyrimidine-2-yl-pipéridine-4-yl)-8-oxa-2,4,5-triaza-tricyclo[9.4.0.0*2,6*]pentadéca-1(11),3,5,12,14-pentaène ;
le 8-chloro-1-(1-pyrimidine-2-yl-pipéridine-4-yl)-4H,6H-5-oxa-2,3,10b-triaza-benzo[e]azulène ;
le 13-chloro-3-(3,4,5,6-tétrahydro-2H-[1,2']bipyridinyl-4-yl)-8-oxa-2,4,5-triaza-tricyclo[9.4.0.0*2,6*]pentadéca-1(11),3,5,12,14-pentaène ;
le dichlorhydrate de 3-(3,4,5,6-tétrahydro-2H-[1,2']bipyridinyl-4-yl)-8-oxa-2,4,5-triazatricyclo[9.4.0.0*2,6*]pentadéca-1(11),3,5,12,14-pentaène ;
le 8-chloro-1-(3,4,5,6-tétrahydro-2H-[1,2']bipyridinyl-4-yl)-4H,6H-5-oxa-2,3,10b-triaza-benzo[e]azulène ;
le dichlorhydrate de 7-chloro-1-(3,4,5,6-tétrahydro-2H-[1,2']bipyridinyl-4-yl)-4H,6H-5-oxa-2,3,10b-triazabenzo[e]-azulène ;
le 1-(3,4,5,6-tétrahydro-2H-[1,2']bipyridinyl-4-yl)-4H,6H-5-oxa-2,3,10b-triaza-benzo[e]azulène ;
le dichlorhydrate de 8-méthoxy-1-(3,4,5,6-tétrahydro-2H-[1,2']bipyridinyl-4-yl)-4H,6H-5-oxa-2,3,10b-triazabenzo[e]-azulène ;
le 8-fluoro-1-(3,4,5,6-tétrahydro-2H-[1,2']bipyridinyl-4-yl)-4H,6H-5-oxa-2,3,10b-triaza-benzo[e]azulène ;
le dichlorhydrate de 8,9-difluoro-1-(3,4,5,6-tétrahydro-2H-[1,2']bipyridinyl-4-yl)-4H,6H-5-oxa-2,3,10b-triaza-benzo[e]-azulène ;
le dichlorhydrate de 9-chloro-1-(3,4,5,6-tétrahydro-2H-[1,2']bipyridinyl-4-yl)-4H,6H-5-oxa-2,3,10b-triazabenzo[e]-azulène ;
le 1-(3,4,5,6-tétrahydro-2H-[1,2']bipyridinyl-4-yl)-8-trifluorométhoxy-4H,6H-5-oxa-2,3,10b-triaza-benzo[e]azulène ;
le 8-méthyl-1-(3,4,5,6-tétrahydro-2H-[1,2']bipyridinyl-4-yl)-4H,6H-5-oxa-2,3,10b-triaza-benzo[e]azulène ; et
la 1-[8-chloro-1-(3,4,5,6-tétrahydro-2H-[1,2']bipyridinyl-4-yl)-4H,6H-2,3,5,10b-tétraaza-benzo[e]azulène-5-yl]-2-diméthylamino-éthanone ;
et leurs sels pharmaceutiquement accepables.

5. Utilisation suivant la revendication 3, dans laquelle le composé de formule (I) est le 8-chloro-5-méthyl-1-(3,4,5,6-tétrahydro-2H-[1,2']bipyridinyl-4-yl)-5,6-dihydro-4H-2,3,5,10b-tétraazo-benzo[e]azulène, le 8-chloro-5-méthyl-1-(1-pyrimidine-2-yl-pipéridine-4-yl)-5,6-dihydro-4H-2,3,5,10b-tétraaza-benzo[e]azulène, ou un de leurs sels pharmaceutiquement acceptables.

6. Utilisation d'un antagoniste du récepteur de vasopressine V1a dans la production d'un médicament destiné au traitement de l'éjaculation précoce ou de l'éjaculation rapide.

7. Utilisation suivant la revendication 6, dans laquelle la CI₅₀ de l'antagoniste du récepteur de vasopressine V1a est inférieure à 100 nM.

8. Utilisation suivant la revendication 7, dans laquelle l'antagoniste du récepteur de vasopressine V1a est sélectif pour les récepteurs de vasopressine V1a.

9. Méthode pour sélectionner des composés utiles pour le traitement de l'éjaculation précoce ou de l'éjaculation rapide, comprenant la sélection des composés pour l'activité antagoniste contre les récepteurs de vasopressine V1a, et la sélection des composés ayant une CI₅₀ inférieure à 100 nM.

10. Utilisation d'une association d'un antagoniste du récepteur de vasopressine V1a et d'un inhibiteur de PDE V pour la préparation d'un médicament destiné au traitement de l'éjaculation précoce ou de l'éjaculation rapide.

11. Utilisation d'une association d'un antagoniste du récepteur de vasopressine V1a et d'un inhibiteur de réabsorption de sérotonine sélectif (SSRI) pour la préparation d'un médicament destiné au traitement de l'éjaculation précoce ou de l'éjaculation rapide.

12. Produits contenant un antagoniste du récepteur de vasopressine V1a et un inhibiteur de PDE V sous forme d'une préparation combinée pour une utilisation simultanée, séparée ou séquentielle dans le traitement de l'éjaculation précoce ou de l'éjaculation rapide.

13. Produits contenant un antagoniste du récepteur de vasopressine V1a et un inhibiteur de réabsorption de sérotonine sélectif (SSRI) pour la préparation d'un médicament destiné au traitement de l'éjaculation précoce ou de l'éjaculation rapide.
